# EUROPEAN PATENT SPECIFICATION

(11) **EP 3 199 639 B1**
(45) Date of publication and mention of the grant of the patent: **30.12.2020**
(21) Application number: 15844093.3
(22) Date of filing: 18.09.2015
(51) Int. Cl.: C12Q 1/48, C12M 1/34, C12N 9/02, C12N 9/04, C12N 9/12, C12Q 1/26, C12Q 1/54, C12Q 1/50, C12Q 1/00

(54) **NOVEL MEASUREMENT METHOD USING KINASE, AND COMPOSITION**
NEUARTIGES MESSVERFAHREN UNTER VERWENDUNG VON KINASE SOWIE ZUSAMMENSETZUNG
NOUVEAU PROCÉDÉ DE MESURE AU MOYEN D'UNE KINASE ET COMPOSITION

(30) Priority: 26.09.2014 JP 2014196720
(43) Date of publication of application: 02.08.2017
(73) Proprietor: Asahi Kasei Pharma Corporation, Chiyoda-ku Tokyo 100-0006 (JP)
(72) Inventor: UEDA, Shigeru, Tokyo 100-0006 (JP); SAKASEGAWA, Shinichi, Tokyo 100-0006 (JP)
(74) Representative: Forstmeyer, Dietmar
(86) International application number: PCT/JP2015/076639
(87) International publication number: WO 2016/047580

(56) References cited:
- EP-A1- 0 881 301
- WO-A1-2010/082665
- WO-A1-2011/056611
- JP-A- S6 047 698
- JP-A- 2002 355 095
- JP-A- 2006 223 163
- JP-A- 2017 038 569
- GROSSMAN G ET AL: "EASTERN GRAY KANGAROO MACROPUS-GIGANTEUS MUSCLE CREATINE KINASE EC-2.7.3.2", AUSTRALIAN JOURNAL OF BIOLOGICAL SCIENCES, vol. 34, no. 3, 1981, pages 269-282, XP009503962, ISSN: 0004-9417
- MATSUOKA T ET AL.: 'An ultrasensitive enzymatic method for measuring mevalonic acid in serum.' J. LIPID RES. vol. 53, no. 9, 2012, pages 1987 - 1992, XP009165622
- ETSURO ITO ET AL.: 'DEVELOPMENT OF SUPER HIGH-SENSITIVE MEASUREMENT OF PROTEINS, Rinsho Byori' THE 57TH CHUGOKU-SHIKOKU REGIONAL CONGRESS SYMPOSIUM: SAISHIN NO RINSHO KENSA UP TO DATE(1) vol. 60, no. 11, 2012, pages 1088 - 1093, XP009501180
- HSU SHU-YI ET AL: "Enzymatic determination of diglyceride using an iridium nano-particle based single use, disposable biosensor.", SENSORS (BASEL, SWITZERLAND) 2010, vol. 10, no. 6, 2010, pages 5758-5773, ISSN: 1424-8220
- GESTA STEPHANE ET AL: "Secretion of a lysophospholipase D activity by adipocytes: Involvement in lysophosphatidic acid synthesis", JOURNAL OF LIPID RESEARCH, vol. 43, no. 6, June 2002 (2002-06), pages 904-910, ISSN: 0022-2275
- "Product Information Glucose-1-Phosphate (G1P) Colorimetric Assay Kit", 1 January 2013 (2013-01-01), Sigma-Aldrich
- ARTISS J D ET AL: "ENZYMIC COLORIMETRIC DETERMINATION OF PHOSPHATIDYL GLYCEROL IN AMNIOTIC FLUID", CLINICAL CHEMISTRY, vol. 30, no. 4, 1984, pages 534-537, ISSN: 0009-9147
- BRINDLEY D N ET AL: "Phosphatidate degradation: Phosphatidate phosphatases (lipins) and lipid phosphate phosphatases", BIOCHIMICA ET BIOPHYSICA ACTA (BBA) - MOLECULAR AND CELL BIOLOGY OF LIPIDS, ELSEVIER, AMSTERDAM, NL, vol. 1791, no. 9, 1 September 2009 (2009-09-01), pages 956-961, XP026500854, ISSN: 1388-1981, DOI: 10.1016/J.BBALIP.2009.02.007 [retrieved on 2009-02-27]
- TEUNISSEN A J ET AL: "COMPARISON OF 5 METHODS FOR DETERMINATION OF 2 3 DI PHOSPHO GLYCERATE IN BLOOD", CLINICAL CHEMISTRY, vol. 20, no. 6, 1974, pages 649-655, ISSN: 0009-9147
- "Product Information 2-Phosphoglycerate Clorimetric/Fluorimetric Assay Kit", 1 January 2013 (2013-01-01), Sigma-Aldrich
- "Asahi Kasei Diagnostic Enzymes", 1 January 2011 (2011-01-01), Asahi Kasei
- DATABASE BIOSIS [Online] BIOSCIENCES INFORMATION SERVICE, PHILADELPHIA, PA, US; 1 July 2003 (2003-07-01), KISHIMOTO TATSUYA ET AL: "A novel colorimetric assay for the determination of lysophosphatidic acid in plasma using an enzymatic cycling method.", Database accession no. PREV200300414784 & CLINICA CHIMICA ACTA, vol. 331, no. 1, 1 July 2003 (2003-07-01), pages 59-67, ISSN: 0009-8981
- Glycolysis by Anonymous

## Description

### Technical Field

The present invention relates to a novel sensitive measuring method employing an enzymatic cycling method using a creatine kinase, and a composition used therefor.

### Background Art

An enzymatic cycling method is a method in which a concentration of a very small amount of a substance is amplified for measurement by utilizing a function of an enzyme. Conventionally, as a method for measuring a coenzyme, a sensitive measuring method for a coenzyme such as nicotinamide adenine dinucleotide (NAD) cycling using two dehydrogenases is known (see, for example, Non Patent Literature 1). Besides, a cycling method for measuring adenosine triphosphate (ATP) similarly using two kinases has been reported (see, for example, Patent Literature 1). On the other hand, examples of an enzymatic cycling method for measuring not a coenzyme but a substrate include a method for performing measurement by using two transferases and a method for performing measurement by using one enzyme.

An example of the method using two enzymes includes a method using transaminase and polyamine oxidase (see, for example, Patent Literature 2). In this method, putrescine transaminase and polyamine oxidase are used for quantitatively determining putrescine through enzymatic cycling reactions using polyamine oxidase for a reaction from putrescine to 4-aminobutanal and using putrescine transaminase for a reverse reaction, and specifically, hydrogen peroxide produced through the polyamine oxidase reaction is measured by a known coloring method. Similarly, a method for measuring a benzylamine using a benzylamine transaminase and a benzylamine oxidase and a method for measuring tyramine using a benzylamine transaminase and a tyramine oxidase have been reported (see, for example, Patent Literatures 3 and 4).

Besides, as a measuring method employing a cycling method using one enzyme, a method using a dehydrogenase is known (see, for example, Patent Literatures 5 and 6). In this method, an enzymatic cycling reaction utilizing reversible reactivity of a dehydrogenase is caused to proceed in the presence of oxidized coenzyme NAD (P) or an analog thereof, and a reduced coenzyme NAD (P) or an analog thereof, so as to sensitively quantitatively determine a substrate for the dehydrogenase, and this method is applied to quantitative determination of bile acid using 3α-hydroxysteroid dehydrogenase in the presence of thio-NAD and reduced NAD, or quantitative determination of glucose-6-phosphate using glucose-6-phosphate dehydrogenase, for example.

Conventionally, as a measuring method for a substrate as a measurement target using a kinase, for example, a method for measuring triglyceride is known (see, for example, Non Patent Literature 2). Besides, a method for measuring creatinine is known (see, for example, Patent Literature 7). In this measuring method, creatinine is converted into creatine by a function of creatinine amidohydrolase, the creatine is further converted, in the presence of ATP, into creatine phosphate and adenosine diphosphate (ADP) by creatine kinase, then the ADP is measured for measuring the creatinine.

### Citation List

### Patent Literature

Patent Literature 1: Japanese Patent Laid-Open No. 2006-223163
Patent Literature 2: Japanese Patent Laid-Open No. 3-180200
Patent Literature 3: Japanese Patent Laid-Open No. 7-155199
Patent Literature 4: Japanese Patent Laid-Open No. 7-177898
Patent Literature 5: Japanese Patent Laid-Open No. 3-224498
Patent Literature 6: Japanese Patent Laid-Open No. 4-335898
Patent Literature 7: Japanese Patent Laid-Open No. 9-285297 and EP 0881301 A1

### Non Patent Literature

Non Patent Literature 1: Seikagaku Jikken Kouza 5 Koso Kenkyuho (jo) (Lecture on Biochemical Experiments 5 Study Method for Enzymes (1)), edited by The Japanese Biochemical Society, Tokyo Kagaku Dojin, 1975, pp. 121-135
Non Patent Literature 2: Koso - Biotechnology eno Shishin - (Enzyme - Guideline for Biotechnology -), edited by Japan Society for Bioscience, Biotechnology, and Agrochemistry, Asakura Publishing Co., Ltd., 1985, pp.97-101

WO 2011/056611 A1 discloses coupled reactions for analysis of nucleotides and their hydrolysis using nucleoside monophosphate kinase (NMPK).

Grossman G and O'Sullivan WJ, "Eastern Grey Kangaroo Muscle Creatine Kinase", Aust. J. Biol. Sci, 34, 269-282 (1981) report the purification and characterization of creatine kinase from the skeletal muscle of the eastern grey kangaroo, *Macropus giganteus.*

### Summary of Invention

### Technical Problem

An object of the present invention as defined in the claims is to provide a method and a composition by which at least one of a creatine kinase forward reaction substrate, a phosphorylated product thereof, and a precursor thereof can be highly accurately measured.

### Solution to Problem

A measuring method employing an enzymatic cycling method is a technique applicable to sensitive quantitative determination by controlling an amount of an enzyme because a signal corresponding to an enzymatic reaction increases in accordance with the amount of the enzyme to be added. In particular, a method using a creatine kinase in an enzymatic cycling method for quantitatively determining a substrate has not yet been known, and measurement targets in the enzymatic cycling method for quantitatively determining a substrate have been limited. Further, a forward reaction usually proceeds priorly to a reverse reaction in a kinase reaction, and it has been regarded that it is technically difficult to apply a kinase to an enzymatic cycling method utilizing a reverse reaction.

On the other hand, as described above, an example of a method for measuring a substrate using a kinase includes the method for measuring creatinine using creatinine kinase (see, for example, Patent Literature 7), the kinase is used merely for a unidirectional stoichiometric conversion reaction, and therefore, if the concentration of a measurement target is low, sufficient sensitivity cannot be always obtained, and hence it is necessary to deal with this problem by, for example, increasing the amount of a specimen. If the amount of a specimen is increased, however, there arises a practical problem in which the measurement is largely affected by a coexisting substance.

Under these circumstances, the present inventors have found the following: a plurality of kinases catalyze both a forward reaction and a reverse reaction of a kinase reaction, and utilize coenzymes having different nucleoside moieties in the forward reaction and the reverse reaction, and when these kinases are used in an enzymatic cycling reaction, a kinase forward reaction substrate and/or a phosphorylated product thereof, or a precursor thereof can be surprisingly sensitively measured, and thus, the present invention was accomplished with the enzyme creatine kinase.

The present invention has the following features:
[1] A measuring method for a kinase forward reaction substrate and/or a phosphorylated product thereof, comprising:
   (1) a step of conducting a cycling reaction according to the following formula (1): 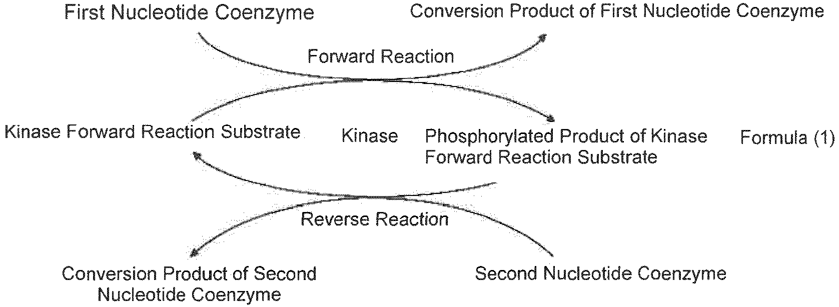 by bringing: (i) a kinase catalyzing a forward reaction for producing a phosphorylated product from a kinase forward reaction substrate and a reverse reaction thereof in the presence of nucleotide coenzymes, the kinase utilizing nucleotide coenzymes at least having different nucleoside moieties in the forward reaction and the reverse reaction, respectively; (ii) a first nucleotide coenzyme of the kinase; and (iii) a second nucleotide coenzyme having a different nucleoside moiety from the first nucleotide coenzyme;
      into contact with a sample;
   (2) a step of detecting an amount of change of a signal corresponding to a change of at least any one of the first nucleotide coenzyme, a conversion product of the first nucleotide coenzyme, the second nucleotide coenzyme, and a conversion product of the second nucleotide coenzyme; and
   (3) a step of calculating, on the basis of the detected change of the signal, an amount of the kinase forward reaction substrate and/or the phosphorylated product thereof contained in the sample;
   wherein the kinase is creatine kinase; and the forward reaction substrate is creatine.
[2] A measuring method for a precursor of a kinase forward reaction substrate, comprising:
   (1) a step of conducting a cycling reaction according to the following formula (1): 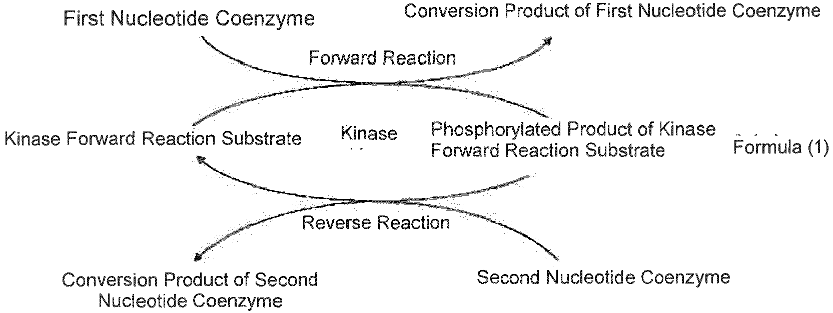 by bringing: (i) a kinase catalyzing a forward reaction for producing a phosphorylated product from a kinase forward reaction substrate and a reverse reaction thereof in the presence of nucleotide coenzymes, the kinase utilizing nucleotide coenzymes at least having different nucleoside moieties in the forward reaction and the reverse reaction, respectively;(ii) a first nucleotide coenzyme of the kinase; and (iii) a second nucleotide coenzyme having a different nucleoside moiety from the first nucleotide coenzyme;
      into contact with a sample having been subjected to a quantitative derivation treatment from the precursor to the kinase forward reaction substrate and/or the phosphorylated product thereof;
   (2) a detection step according to step (2) of claim 1; and
   (3) a step of calculating, on the basis of the detected change of the signal, an amount of the precursor contained in the sample;
   wherein the kinase is creatine kinase; the forward reaction substrate is creatine; and the precursor is creatinine.
[3] The measuring method according to [2] described above, wherein creatinine is brought into contact with creatinine amidohydrolase (EC 3.5.2.10) in the presence of water in the quantitative derivation treatment from the precursor to the kinase forward reaction substrate and/or the phosphorylated product thereof.
[4] The measuring method according to any one of [1] to [3] described above, wherein the nucleoside moiety of the first nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.
[5] The measuring method according to any one of [1] to [4] described above,
   wherein the nucleoside moiety of the second nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.
[6] The measuring method according to any one of [1] to [5] described above,
   wherein a combination of the nucleoside moiety of the first nucleotide coenzyme and the nucleoside moiety of the second nucleotide coenzyme is a combination of adenosine and inosine, guanosine and adenosine, deoxyadenosine and guanosine, deoxyadenosine and deoxyguanosine, deoxyadenosine and inosine, or inosine and adenosine.
[7] The measuring method according to any one of [1] to [6] described above,
   wherein the nucleoside moiety of the first nucleotide coenzyme is adenosine, and the nucleoside moiety of the second nucleotide coenzyme is inosine.
[8] The measuring method according to any one of [1] to [7] described above,
   wherein the first nucleotide coenzyme is adenosine triphosphate (ATP), and the second nucleotide coenzyme is inosine diphosphate (IDP).
[9] The measuring method according to any one of [1] to [8] described above,
   wherein in the step of detecting the change of the signal, an amount of change of a signal corresponding to an increased amount of the conversion product of the first nucleotide coenzyme resulting from the forward reaction or the conversion product of the second nucleotide coenzyme resulting from the reverse reaction is detected by using a detection enzyme able to utilize the conversion product of the first nucleotide coenzyme resulting from the forward reaction but unable to utilize the second nucleotide coenzyme, or a detection enzyme unable to utilize the first nucleotide coenzyme but able to utilize the conversion product of the second nucleotide coenzyme resulting from the reverse reaction.
[10] The measuring method according to any one of [1] to [9] described above,
   wherein in the step of detecting the change of the signal, an amount of change of a signal varied in accordance with an increased amount of adenosine diphosphate (ADP) is detected by using adenosine diphosphate (ADP)-dependent glucokinase (EC 2.7.1.147) in the presence of glucose.
[11] The measuring method according to any one of [1] to [10] described above,
   wherein in the step of detecting the amount of change of the signal, an amount of change of a signal varied in accordance with an increased amount of adenosine diphosphate (ADP) is detected by using adenosine diphosphate (ADP)-dependent glucokinase (EC 2.7.1.147) in the presence of any one of coenzymes of thionicotinamide adenine dinucleotide phosphate (thio-NADP), thionicotinamide adenine dinucleotide (thio-NAD), nicotinamide adenine dinucleotide phosphate (NADP) and nicotinamide adenine dinucleotide (NAD), glucose, and glucose 6-phosphate dehydrogenase.
[12] A reagent kit for measuring a kinase forward reaction substrate and/or a phosphorylated product thereof, comprising:
   (a) a kinase that catalyzes a forward reaction for producing a phosphorylated product from the kinase forward reaction substrate and a reverse reaction thereof in the presence of nucleotide coenzymes, and utilizes nucleotide coenzymes at least having different nucleoside moieties in the forward reaction and the reverse reaction, respectively,
   (b) a first nucleotide coenzyme for the forward reaction; and
   (c) a second nucleotide coenzyme for the reverse reaction having a different nucleoside moiety from the first nucleotide coenzyme,
   wherein the kinase is creatine kinase; and the kinase forward reaction substrate is creatine.
[13] A reagent kit for measuring a precursor of a kinase forward reaction substrate, and used after the precursor is subjected to a quantitative derivation treatment from the precursor to the kinase forward reaction substrate and/or the phosphorylated product thereof, comprising components (a), (b) and (c) of [12];
   wherein the kinase is creatine kinase; the kinase forward reaction substrate is creatine; and the precursor is creatinine; and
   wherein creatine is to be derived by bringing creatinine into contact with creatinine amidohydrolase (EC 3.5.2.10) in the presence of water in the quantitative derivation treatment from the precursor to the kinase forward reaction substrate and/or the phosphorylated product thereof.
[14] The reagent kit according to [12] or [13] described above,
   wherein the nucleoside moiety of the first nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.
[15] The reagent kit according to any one of [12] to [14] described above,
   wherein the nucleoside moiety of the second nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.
[16] The reagent kit according to any one of [12] to [15] described above,
   wherein a combination of the nucleoside moiety of the first nucleotide coenzyme and the nucleoside moiety of the second nucleotide coenzyme is a combination of adenosine and inosine, guanosine and adenosine, deoxyadenosine and guanosine, deoxyadenosine and deoxyguanosine, deoxyadenosine and inosine, or inosine and adenosine.
[17] The reagent kit according to any one of [12] to [16] described above,
   wherein the nucleoside moiety of the first nucleotide coenzyme is adenosine, and the nucleoside moiety of the second nucleotide coenzyme is inosine.
[18] The reagent kit according to any one of [12] to [17] described above,
   wherein the first nucleotide coenzyme is adenosine triphosphate (ATP), and the second nucleotide coenzyme is inosine diphosphate (IDP).
[19] The reagent kit according to any one of [12] to [17] described above, further comprising a detection enzyme able to utilize a conversion product of the first nucleotide coenzyme resulting from the forward reaction but unable to utilize the second nucleotide coenzyme, or a detection enzyme unable to utilize the first nucleotide coenzyme but able to utilize a conversion product of the second nucleotide coenzyme resulting from the reverse reaction.
[20] The reagent kit according to [19] described above, wherein the detection enzyme is adenosine diphosphate (ADP)-dependent glucokinase (EC 2.7.1.147), and the composition further comprises glucose.
[21] The reagent kit according to [19] described above, further comprising thionicotinamide adenine dinucleotide phosphate (thio-NADP), thionicotinamide adenine dinucleotide (thio-NAD), nicotinamide adenine dinucleotide phosphate (NADP) or nicotinamide adenine dinucleotide (NAD); and glucose-6-phosphate dehydrogenase.
[22] The reagent kit according to [13] or [14] described above,
   wherein the measurement target is creatinine as the precursor,
   the reagent kit is to be used after a quantitative derivation treatment from the creatinine to creatine by bringing the creatinine into contact with creatinine amidohydrolase (EC 3.5.2.10) in the presence of water, and comprises:
   (a) creatine kinase;
   (b) adenosine triphosphate (ATP);
   (c) inosine diphosphate (IDP);
   (d) adenosine diphosphate (ADP)-dependent glucokinase (EC 2.7.1.147);
   (e) glucose;
   (f) thionicotinamide adenine dinucleotide phosphate (thio-NADP), thionicotinamide adenine dinucleotide (thio-NAD), nicotinamide adenine dinucleotide phosphate (NADP) or nicotinamide adenine dinucleotide (NAD); and
   (g) glucose-6-phosphate dehydrogenase.
[23] A composition comprising:
   (a) a kinase that catalyzes a forward reaction for producing a phosphorylated product from a kinase forward reaction substrate and a reverse reaction thereof in the presence of nucleotide coenzymes, and utilizes nucleotide coenzymes at least having different nucleoside moieties in the forward reaction and the reverse reaction, respectively,
   (b) a first nucleotide coenzyme for the forward reaction; and
   (c) a second nucleotide coenzyme for the reverse reaction having a different nucleoside moiety from the first nucleotide coenzyme,
   wherein the kinase is creatine kinase; and the kinase forward reaction substrate is creatine.

### Advantageous Effects of Invention

According to the present invention, at least one of the kinase substrate creatine, a phosphorylated product thereof, and the precursor creatinine thereof can be highly sensitively measured.

According to the present invention, creatinine, which has been difficult to highly sensitively measure by a conventional cycling method, can be highly sensitively measured.

### Brief Description of Drawings

[Figure 1] Figure 1 is a graph illustrating the correlation between a creatine concentration in a sample and an absorbance change obtained by using a creatine kinase.
[Figure 2] Figure 2 is a graph illustrating the correlation between a creatine concentration in a sample and an absorbance change obtained by using a creatine kinase in an example in which deoxyinosine diphosphate (IDP) was contained in a reaction solution and in a comparative example in which IDP was not contained in a reaction solution.
[Figure 3] Figure 3 is a graph illustrating the correlation between a creatinine concentration in a sample and an absorbance change obtained by using a creatine kinase.
[Figure 12] Figure 12 is a graph illustrating the correlation between a creatinine concentration in a sample and an absorbance change obtained by using a creatine kinase.

### Description of Embodiment

Now, an embodiment for practicing the present invention (hereinafter also referred to as the "present embodiment") will be specifically described. It is noted that EC numbers (Enzyme Commission numbers) mentioned herein are those confirmed as of September 12, 2014.

A method of the present embodiment is: (A) a measuring method for a kinase forward reaction substrate and/or a phosphorylated product thereof according to [1] described above; or (B) a measuring method for a precursor of a kinase forward reaction substrate according to [2] described above; including:
(1) a step of conducting an enzymatic cycling reaction represented by the following formula (3): 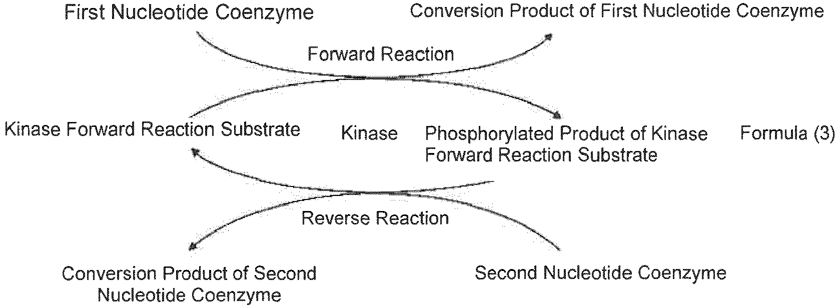 by bringing a kinase catalyzing a forward reaction for producing the phosphorylated product from the kinase forward reaction substrate and a reverse reaction thereof in the presence of nucleotide coenzyme, the kinase utilizing nucleotide coenzymes at least having different nucleoside moieties in the forward reaction and the reverse reaction, respectively, a first nucleotide coenzyme of the kinase and a second nucleotide coenzyme having a different nucleoside moiety from the first nucleotide coenzyme
   into contact with a sample in case of method (A) where the measurement target is a precursor; or into contact with a sample having been subjected to a quantitative derivation treatment from the precursor to the kinase forward reaction substrate and/or the phosphorylated product thereof in case of method (B) where the measurement target is a precursor;
(2) a step of detecting an amount of change of a signal corresponding to a change of at least any one of the first nucleotide coenzyme, a conversion product of the first nucleotide coenzyme, the second nucleotide coenzyme, and a conversion product of the second nucleotide coenzyme; and
(3) a step of calculating, on the basis of the detected change of the signal, an amount of the kinase forward reaction substrate and/or the phosphorylated product thereof in case of method (A); or an amount of the precursor in case of method (B); contained in the sample;
wherein the kinase is creatine kinase; the forward reaction substrate is creatine; and the precursor is creatinine.

The kinase used in the method of the present embodiment is, as shown in the formula (3), a kinase capable of catalyzing, in the presence of the first nucleotide coenzyme, the forward reaction for producing the phosphorylated product thereof from the forward reaction substrate, and catalyzing, in the presence of the second nucleotide coenzyme, the reverse reaction for producing the forward reaction substrate from the phosphorylated product of the forward reaction substrate.

In the method of the present embodiment, the sample to be tested for the amount, contained therein, of the kinase forward reaction substrate and/or the phosphorylated product thereof, or the precursor thereof is not especially limited, and is, for example, a biological sample of a human or an animal, preferably human blood, and further preferably human serum. It should be noted that it may be unknown before performing the method whether or not the sample contains the measurement target, such as the kinase forward reaction substrate and/or the phosphorylated product thereof, or the precursor thereof. The method of the present embodiment can be performed to reveal that the sample does not contain the kinase forward reaction substrate and/or the phosphorylated product thereof, or the precursor thereof.

In the method of the present embodiment, the "precursor" refers to a substance from which to the kinase forward reaction substrate and/or the phosphorylated product thereof is derived. If there are a plurality of reaction steps to obtain the kinase forward reaction substrate and/or the phosphorylated product thereof, the precursor embraces a starting material thereof and all intermediates, and can be any of these.

In the method of the present embodiment, the "quantitative derivation treatment" refers to a treatment for deriving, from the precursor, the kinase forward reaction substrate and/or the phosphorylated product thereof in such a manner that a decreased amount of the precursor corresponds to an increased amount of the kinase forward reaction substrate and/or the phosphorylated product thereof those are derived products. Specifically, it can be any treatment by which the amount of the precursor can be calculated by the method of the present embodiment on the basis of an amount of change of a signal corresponding to a change of at least any one of the first nucleotide coenzyme, the conversion product of the first nucleotide coenzyme, the second nucleotide coenzyme, and the conversion product of the second nucleotide coenzyme. Examples of the quantitative derivation treatment include, but are not limited to, known chemical treatments. Examples of the chemical treatments include, but are not limited to, treatment using chemical reactions such as hydrolysis, oxidation and reduction. A specific example includes hydrolysis using an acid or an alkali.

Examples of the treatment, excluding the chemical treatments, include enzymatic treatments. For example, from creatinine, creatine can be derived by bringing it into contact with creatinine amidohydrolase (creatininase) (EC 3.5.2.10) in the presence of water. Accordingly, in the method of the present embodiment, if creatine is measured by using creatine kinase as the kinase, creatinine, that is, a precursor of creatine, can be measured.

The kinase usable in the method of the present embodiment of the disclosure is not especially limited as long as it is capable of catalyzing, in the presence of nucleotide coenzymes, the forward reaction for producing a phosphorylated product from a kinase forward reaction substrate, and the reverse reaction thereof, and has a characteristic that nucleotide coenzymes at least having different nucleoside moieties can be used in the forward reaction and the reverse reaction, respectively. Here, the "kinase forward reaction" performed in the method of the present embodiment refers to a reaction in a direction to transfer phosphoric acid from a nucleotide coenzyme to a forward reaction substrate among high-energy phosphotransfers catalyzed by a kinase, and the number of phosphoric acids to be transferred is not limited. Preferably, the number of phosphoric acids to be transferred is one. Besides, the "kinase reverse reaction" refers to a reaction in a reverse direction to the "kinase forward reaction".

In the present embodiment, if the measurement target is the kinase forward reaction substrate and/or the phosphorylated product thereof, examples of a combination of the measurement target and the kinase include, but are not limited to, the followings. It is noted that each of the following combinations is described as a measurement target: a kinase in this order. In addition, a phosphorylated product thereof refers to a phosphorylated product corresponding to the described kinase forward reaction substrate.

Creatine and/or a phosphorylated product thereof: creatine kinase

Preferable examples include, but are not limited to, the following combinations each including a kinase specified by EC number (Enzyme Commission number).

Creatine and/or a phosphorylated product thereof: creatine kinase (EC 2.7.3.2)

If the measurement target is the precursor, examples of each combination of the measurement target, the kinase and the kinase forward reaction substrate and/or the phosphorylated product thereof include, but are not limited to, the followings. It is noted that each of the following combinations is described as a measurement target: a kinase: a kinase forward reaction substrate and/or the phosphorylated product thereof in this order.

Creatinine: creatine kinase: creatine and/or a phosphorylated product thereof

Preferable examples include, but are not limited to, the following combinations each including a kinase specified by EC number:
Creatinine: creatine kinase (EC 2.7.3.2): creatine and/or a phosphorylated product thereof

The "nucleotide coenzyme" used in the method of the present embodiment refers to a phosphorylated product of nucleoside working as a coenzyme of a kinase. The first nucleotide coenzyme and the second nucleotide coenzyme used in the method of the present embodiment are nucleotide coenzymes having different nucleoside moieties from each other. The type of the nucleoside moiety of the nucleotide coenzyme used in the method of the present embodiment is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Besides, phosphorylated products of these may be a monophosphorylated product, a diphosphorylated product or a triphosphorylated product.

Examples of the nucleotide coenzyme include ribonucleotide and deoxyribonucleotide.

Specific examples of the ribonucleotide used as the nucleotide coenzyme include, but are not limited to, ATP, ADP, adenylate (AMP), guanosine triphosphate (GTP), guanosine diphosphate (GDP), guanylate (GMP), 5-methyluridine triphosphate (TTP), 5-methyluridine diphosphate (TDP), 5-methyluridine monophosphate (TMP), uridine triphosphate (UTP), uridine diphosphate (UDP), uridine monophosphate (UMP), cytidine triphosphate (CTP), cytidine diphosphate (CDP), cytidine monophosphate (CMP), xanthosine triphosphate (XTP), xanthosine diphosphate (XDP), xanthosine monophosphate (XMP), inosine triphosphate (ITP), inosine diphosphate (IDP) and inosine monophosphate (IMP).

Besides, specific examples of the deoxyribonucleotide used as the nucleotide coenzyme include, but are not limited to, deoxyadenosine triphosphate (dATP), deoxyadenosine diphosphate (dADP), deoxyadenosine monophosphate (dAMP), deoxyguanosine triphosphate (dGTP), deoxyguanosine diphosphate (dGDP), deoxyguanosine monophosphate (dGMP), thymidine triphosphate (dTTP), thymidine diphosphate (dTDP), thymidine monophosphate (dTMP), deoxyuridine triphosphate (dUTP), deoxyuridine diphosphate (dUDP), deoxyuridine monophosphate (dUMP), deoxycytidine triphosphate (dCTP), deoxycytidine diphosphate (dCDP), deoxycytidine monophosphate (dCMP), deoxyxanthosine triphosphate (dXTP), deoxyxanthosine diphosphate (dXDP), deoxyxanthosine monophosphate (dXMP), deoxyinosine triphosphate (dITP), deoxyinosine diphosphate (dIDP) and deoxyinosine monophosphate (dIMP).

Preferable examples of the nucleotide coenzyme include, but are not limited to, ATP, ADP, GTP, GDP, TTP, TDP, UTP, UDP, CTP, CDP, XTP, XDP, ITP, IDP, dATP, dADP, dGTP, dGDP, dTTP, dTDP, dUTP, dUDP, dCTP, dCDP, dXTP, dXDP, dITP and dIDP.

To such a nucleotide enzyme, for example, a substituent having coloring ability or the like may be bonded in some cases.

The nucleoside moiety of the first nucleotide coenzyme used in the forward reaction in the method of the present embodiment is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Preferable examples include adenosine, inosine, guanosine and deoxyadenosine. A more preferable example includes adenosine. Besides, the phosphorylated products of these may be a monophosphorylated product, a diphosphorylated product or a triphosphorylated product, and a triphosphorylated product is preferred. Specific examples of the first nucleotide coenzyme include the aforementioned specific examples, and further preferable examples include ATP, GTP, TTP, UTP, CTP, XTP, ITP, dATP, dGTP, dTTP, dUTP, dCTP, dXTP and dITP.

The nucleoside moiety of the second nucleotide coenzyme used in the reverse reaction in the method of the present embodiment is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Preferable examples include inosine, guanosine, adenosine and deoxyguanosine. A more preferable example includes inosine. Besides, the phosphorylated products of these may be a monophosphorylated product, a diphosphorylated product or a triphosphorylated product, and a diphosphorylated product is preferred. Specific examples of the second nucleotide coenzyme include the aforementioned specific examples, and further preferable examples include ADP, GDP, TDP, UDP, CDP, XDP, IDP, dADP, dGDP, dTDP, dUDP, dCDP, dXDP and dIDP.

Preferable nucleoside moieties of the first nucleotide coenzyme and the second nucleotide coenzyme used in the method of the present embodiment are not especially limited, and examples include adenosine, inosine, guanosine or deoxyadenosine, and the nucleoside moieties are different from each other. Examples of the combination of the nucleoside moiety of the first nucleotide coenzyme and the nucleoside moiety of the second nucleotide coenzyme include combinations of adenosine and inosine, guanosine and adenosine, deoxyadenosine and guanosine, deoxyadenosine and deoxyguanosine, deoxyadenosine and inosine, or inosine and adenosine. More preferably, the nucleoside moiety of the first nucleotide coenzyme is adenosine, and the nucleoside moiety of the second nucleotide coenzyme is inosine.

In the method of the present embodiment, the "conversion product of the first nucleotide coenzyme resulting from the forward reaction" refers to a dephosphorylated product of the first nucleotide coenzyme obtained in the kinase forward reaction after phosphoric acid is transferred from the first nucleotide coenzyme to the kinase forward reaction substrate. Besides, the "conversion product of the second nucleotide coenzyme resulting from the reverse reaction" refers to a phosphorylated product of the second nucleotide coenzyme obtained in the kinase reverse reaction after phosphoric acid is transferred from the kinase reverse reaction substrate to the second nucleotide coenzyme. Here, the number of phosphoric acids to be transferred may be one, two, or three. The number is preferably one.

In the step (1) of the method of the present embodiment, the cycling reaction of the formula (3) is caused, for example, as follows:
For example, the first and second nucleotide coenzymes are prepared in an excessive amount, specifically, 0.1 mmol/L or more and 12 mmol/L or less, more preferably 0.2 mmol/L to 6 mmol/L, and to the resultant, a pH buffer solution for retaining pH during a reaction is added in a concentration of 20 mmol/L or more and 200 mmol/L or less to obtain a reaction solution. The pH during the reaction may be appropriately selected as a condition for conducting the reaction to efficiently proceed, and is usually adjusted to around neutral of pH 5.5 or more and pH 8.5 or less, and a metal salt such as magnesium chloride, that is, a kinase activating agent, is added in a concentration of 0.5 mmol/L or more and 10 mmol/L or less. To the resultant, the aforementioned kinase is added, the reaction solution is precedently heated to 25°C or more and 42°C or less, more preferably to the vicinity of 37°C, and the forward reaction substrate and/or the phosphorylated product thereof in a smaller amount as compared with the concentration of the first and second nucleotide coenzymes was added to start the reaction.

The amount of the enzyme to be added will now be described. In a general enzymatic cycling reaction, the cycling rate depends on the amount of an enzyme, and it is known that as the Michaelis constant (the Km value) to a substrate is smaller, higher sensitivity can be attained with a smaller amount of the enzyme. Therefore, as a rough standard of the amount of the enzyme to be added, an amount corresponding to an enzyme unit (unit: u) equal to or more of, and preferably twice as much as or more of, a larger Km value (mmol/L) between those of the forward reaction substrate and the phosphorylated product thereof is added per 1 mL of the reaction solution, and as for the upper limit, an amount that can be substantially added may be added. A Km value can be obtained also by a known method. It is noted that the amounts mentioned herein are given merely as examples and are not restrictive.

Hereinafter, the description will be made on the assumption of a creatine kinase (CK). The CK is an enzyme widely present in the animal kingdom, and is abundantly contained in skeletal muscles, heart muscles, brains and the like. CKs derived from rabbits, cows, pigs, chickens and the like are commercially available, and information on the specificity of nucleotide coenzymes can be obtained from literatures. With respect to, for example, a rabbit muscle CK, it has been reported that IDP shows relative activity of 29% assuming that the activity of ADP is 100% (J Biol Chem., 241, 3116-3125, 1966). Besides, when hydrogen peroxide is produced, by using creatine amidohydrolase (EC 3.5.3.3.) and sarcosine oxidase (EC 1.5.3.1), from creatine, which is produced, for example, by using nucleoside diphosphate excluding ADP through a reverse reaction using creatine phosphate as a substrate, and the thus produced hydrogen peroxide is quantitatively determined, the specificity of the CK to the nucleoside diphosphate excluding ADP can be easily confirmed. For example, the CK derived from rabbit muscle has high specificity to IDP, and hence, ATP can be selected as the first nucleotide coenzyme used in the forward reaction, and IDP can be selected as the second nucleotide used in the reverse reaction.

In order to perform the cycling reaction of the method of the present embodiment, the pH dependency may be examined in the presence of the two nucleotide coenzymes, so as to appropriately select pH suitable for efficient proceeding of the cycling reaction. For example, with respect to a CK derived from rabbit muscle or human muscle, a buffer solution around neutral of pH 6 or more and pH 8 or less may be optimally used in some cases. Then, in the presence of excessive amounts, more specifically, 0.1 mmol/L or more and 12 mmol/L or less, preferably 0.2 mmol/L or more and 6 mmol/L or less, of ADP and IDP, creatine or creatine phosphate in a smaller amount than the ADP and the IDP, and enzyme CK may be added. With respect to the additive amount of the enzyme, it has been reported that the Km value of, for example, the CK derived from rabbit muscle to creatine and creatine phosphate are respectively 16 mmol/L and 5 mmol/L (J Biol Chem. 210, p. 65, 1954). In accordance with the above-described rough standard of the additive amount of the enzyme, these values respectively correspond to 16 u/mL and 32 u/mL. Accordingly, the enzyme may be added in a concentration of preferably 32 u/mL or more with an upper limit of about 800 u/mL, but these are not restrictive.

In the step (2) of the method of the present embodiment, an amount of change of a signal corresponding to a change of any one of the first nucleotide coenzyme, the conversion product of the first nucleotide coenzyme resulting from the forward reaction, the second nucleotide coenzyme, and the conversion product of the second nucleotide coenzyme resulting from the reverse reaction may be detected. Besides, the change of the signal may be a decreased amount of the first nucleotide coenzyme or the second nucleotide coenzyme, or an increased amount of the conversion product of the first nucleotide coenzyme resulting from the forward reaction or the conversion product of the second nucleotide coenzyme resulting from the reverse reaction. Preferably, it is the increased amount of the conversion product of the first nucleotide coenzyme resulting from the forward reaction or the conversion product of the second nucleotide coenzyme resulting from the reverse reaction. As a method for detecting the change, any of known methods such as the HPLC can be employed. If the HPLC is employed, after terminating the enzymatic reaction by adding a chelating agent or the like, the conversion product of the first nucleotide resulting from the forward reaction or the conversion product of the second nucleotide coenzyme resulting from the reverse reaction may be appropriately selected to be quantitatively determined by using a reverse phase column or the like. Such information on the HPLC analysis is easily available from resin manufacturers.

Besides, in the step (2) of the method of the present embodiment, a detection enzyme able to utilize the conversion product of the first nucleotide coenzyme resulting from the forward reaction but unable to utilize the second nucleotide coenzyme may be used for detecting an amount of change of a signal varied in accordance with the increased amount of the conversion product of the first nucleotide coenzyme resulting from the forward reaction. Alternatively, a detection enzyme unable to utilize the first nucleotide coenzyme but able to utilize the conversion product of the second nucleotide coenzyme resulting from the reverse reaction may be used for detecting an amount of change of a signal varied in accordance with the increased amount of the conversion product of the second nucleotide coenzyme resulting from the reverse reaction.

For example, a method in which an amount of change of a signal corresponding to the increased amount of ADP is detected in the presence of glucose by using adenosine diphosphate-dependent glucokinase (ADP-dependent glucokinase, EC 2.7.1.147) may be employed. It is known that ADP-dependent glucokinase derived from Pyrococcus furiosus can utilize, as the nucleotide coenzyme, CDP in addition to ADP, but works merely slightly on GDP and IDP. Accordingly, if the ADP-dependent glucokinase is caused to coexist as a detection enzyme in the kinase cycling reaction using ATP as the first nucleotide coenzyme and the IDP or GDP as the second nucleotide coenzyme, ADP, that is, the conversion product of the first nucleotide coenzyme resulting from the forward reaction, alone can be specifically detected. This is actually confirmed as described later.

Besides, it has been reported that glycerol kinase derived from E. coli uses ATP alone as a phosphate donor. Accordingly, in a case where for example, a kinase using ITP, GTP or CTP as the first nucleotide coenzyme, and using ADP as the second nucleotide coenzyme is used in the method of the present embodiment, if glycerol kinase derived from E. coli is used as a detection enzyme, ATP alone can be specifically detected. As the detection enzyme for specifically detecting ATP alone, other kinases specific to ATP alone can be used in addition to the glycerol kinase derived from E. coli, and the specificity of nucleotide coenzymes may be appropriately selected with reference to literature information. Besides, a large number of enzymes out of synthetic enzymes belonging to the enzyme commission number E 6 have been reported to specifically detect ATP alone, and hence, such enzymes can be used as the detection enzyme in a detection reaction. For example, it has been reported that NAD synthetase derived from E. coli (EC 6.3.1.5) does not work on nucleotide coenzymes excluding ATP. In this manner, in the step (2) of the method of the present embodiment, the detection enzyme to be usable may be appropriately selected in consideration of the combination of the first nucleotide coenzyme and the second nucleotide coenzyme of the kinase used in the method of the present embodiment. There is, however, a possibility that the specificity to a coenzyme depends not only on the origin of the enzyme but also on other conditions such as the additive amounts of the coenzymes and the enzyme. Therefore, a result different from that reported in a literature may be obtained in some cases, and hence, the specificity may be actually confirmed for the selection.

Preferably, the adenosine diphosphate-dependent glucokinase (EC 2.7.1.147) is used as the detection enzyme. If the ADP-dependent glucokinase is used, a product, that is, AMP or glucose-6-phosphate, can be quantitatively determined by a known method. In some cases, the glucose-6-phosphate can be used for detecting an amount of change of a signal varied in accordance with the increased amount of ADP in the presence of a coenzyme of any one of thio-NADP, thio-NAD, NADP and NAD, glucose and glucose-6-phosphate dehydrogenase. For example, the glucose-6-phosphate can be measured, in the presence of NAD (P) using glucose-6-phosphate dehydrogenase (G6PDH), as an absorbance change at 340 nm based on reduced NAD (P). Alternatively, instead of NAD (P), a coenzyme analog such as thio-NAD (P) can be used.

In the measurement of the reduced NAD (P), quantitative determination can be made also by using an electron carrier of 1-methoxy PMS in the presence of a hydrogen acceptor, such as nitrotetrazolium blue (Nitro-NB), 2-(4-iodophenyl)-3-(4-nitrophenyl)-5-(2,4-disulfophenyl)-2H-tetrazolium,sodium salt (WST-1), and 2-(4-iodophenyl)-3-(2,4-dinitrophenyl)-5-disulfophenyl)-2H-tetrazolium,sodium salt (WST-3), or by measuring an absorbance of a visible portion as a formazan dye using the enzyme diaphorase. Such compounds can be easily purchased. Besides, it can be detected by using a fluorescent reagent or a luminescent reagent in combination. Alternatively, glucose-6-phosphate can be highly sensitively quantitatively determined by an enzymatic cycling method using G6PDH (see, for example, Patent Literature 7). The step of detecting changes of these varying signals can be performed simultaneously with or separately from the cycling reaction performed in the method of the present embodiment. Similarly, also when glycerol kinase derived from E. coli is used, a product, that is, glycerol-3-phosphate, can be quantitatively determined by a known method.

It should be noted that, although the method of the present embodiment can be performed by using one kinase, the cycling reaction can be carried out using two or more enzymes different in origin but catalyzing the same reaction.

In the method of the present embodiment, as a method for calculating the amount of the kinase forward reaction substrate and/or the phosphorylated product thereof, or the precursor thereof contained in the sample, any of known methods can be employed. For example, in the enzymatic cycling reaction, the conversion product of the first nucleotide coenzyme resulting from the forward reaction and the conversion product of the second nucleotide coenzyme resulting from the reverse reaction tend to increase in proportion to the time. In order to calculate the amount of a test substance in a sample, a reaction time of the enzymatic cycling reaction is specified (to, for example, minute 5 to minute 7 after starting the reaction), and with a substance having a known concentration used as a reference (a calibrator) set as a control, an amount of change of a signal corresponding to a conversion product of the calibrator is measured, and thus, the amount of the test substance in the sample can be calculated. As the calibrator, the kinase forward reaction substrate or the phosphorylated product thereof can be used.

If there is one or a plurality of steps of introducing the precursor to the kinase forward reaction substrate and/or the phosphorylated product of the forward reaction substrate, the enzymatic cycling reaction may be performed after performing the step of introducing the precursor to the kinase forward reaction substrate and/or the phosphorylated product of the forward reaction substrate. Besides, in a case where the step of introducing the precursor to the kinase forward reaction substrate and/or the phosphorylated product of the forward reaction substrate is an enzymatic reaction, it can be introduced to the kinase forward reaction substrate and/or the phosphorylated product of the forward reaction substrate in a short time by adding an excessive amount of an enzyme catalyzing the derivation reaction, and therefore, the cycling reaction of the method of the present embodiment can be performed with the kinase precedently contained. In this case, a prescribed time after completing the derivation reaction may be specified as the reaction time.

In a case where the forward reaction substrate and the phosphorylated product thereof are mixedly contained in the sample, if merely one of the forward reaction substrate and the phosphorylated product is desired to be quantitatively determined, for example, a treatment for converting the substance not to be measured into a different substance may be performed by a known method before the enzymatic cycling reaction.

Further, the present embodiment provides a measuring composition for at least one of a kinase forward reaction substrate, a phosphorylated product thereof, and a precursor thereof; and in particular: (A) a reagent kit for measuring a kinase forward reaction substrate and/or a phosphorylated product thereof according to [12] described above; or (B) a reagent kit for measuring a precursor of a kinase forward reaction substrate according to [13] described above.

In a case where the measurement target by the composition according to the present embodiment is the kinase forward reaction substrate and/or the phosphorylated product thereof, the measurement target and the kinase include the followings. It is noted that each of the following combinations is described as a measurement target: a kinase in this order. In addition, a phosphorylated product thereof refers to a phosphorylated product corresponding to the described kinase forward reaction substrate.
Creatine and/or a phosphorylated product thereof: creatine kinase

Preferable examples include, but are not limited to, the following combinations each including a kinase specified by EC number.
Creatine and/or a phosphorylated product thereof: creatine kinase (EC 2.7.3.2)

In a case where the measurement target is the precursor, the measurement target, the kinase and the kinase forward reaction substrate and/or the phosphorylated product thereof include the followings. It is noted that each of the following combinations is described as a measurement target: a kinase: a kinase forward reaction substrate and/or the phosphorylated product thereof in this order.
Creatinine: creatine kinase: creatine and/or a phosphorylated product thereof

Preferable examples include, but are not limited to, the following combinations each including a kinase specified by EC number.
Creatinine: creatine kinase (EC 2.7.3.2): creatine and/or a phosphorylated product thereof

In the composition according to the present embodiment, in a case where the measurement target is the kinase forward reaction substrate and/or the phosphorylated product thereof, each combination of the measurement target and the kinase can be determined in the same manner as described above with respect to the method of the present embodiment. Further, also if the measurement target is the precursor, each combination of the measurement target, the kinase, and the kinase forward reaction substrate and/or the phosphorylated product thereof can be determined in the same manner as described above with respect to the method of the present embodiment.

The first nucleotide coenzyme and the second nucleotide coenzyme are nucleotide coenzymes having different nucleoside moieties from each other. The type of the nucleoside moiety is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Further, phosphorylated products of these may be a monophosphorylated product, a diphosphorylated product or a triphosphorylated product.

Specific examples of the nucleotide coenzymes include, but are not limited to, ATP, ADP, AMP, GTP, GDP, GMP, TTP, TDP, TMP, UTP, UDP, UMP, CTP, CDP, CMP, XTP, XDP, XMP, ITP, IDP, IMP, dATP, dADP, dAMP, dGTP, dGDP, dGMP, dTTP, dTDP, dTMP, dUTP, dUDP, dUMP, dCTP, dCDP, dCMP, dXTP, dXDP, dXMP, dITP, dIDP and dIMP. Preferable examples include ATP, ADP, GTP, GDP, TTP, TDP, UTP, UDP, CTP, CDP, XTP, XDP, ITP, IDP, dATP, dADP, dGTP, dGDP, dTTP, dTDP, dUTP, dUDP, dCTP, dCDP, dXTP, dXDP, dITP and dIDP. To such a nucleotide enzyme, for example, a substituent having coloring ability or the like may be bonded in some cases.

The nucleoside moiety of the first nucleotide coenzyme for the forward reaction used in the composition of the present embodiment is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Preferable examples include adenosine, inosine, guanosine, deoxyadenosine and deoxyadenosine. A more preferable example includes adenosine. Besides, the phosphorylated products of these may be a monophosphorylated product, a diphosphorylated product or a triphosphorylated product, and a triphosphorylated product is preferred. Specific examples of the first nucleotide coenzyme include the aforementioned specific examples, and further preferable examples include ATP, GTP, TTP, UTP, CTP, XTP, ITP, dATP, dGTP, dTTP, dUTP, dCTP, dXTP and dITP.

The nucleoside moiety of the second nucleotide coenzyme for the reverse reaction used in the composition of the present embodiment is not especially limited, and examples include adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine. Preferable examples include inosine, guanosine, adenosine and deoxyguanosine. A more preferable example includes inosine. Besides, the phosphorylated products of these may be a monophosphorylated product, a diphosphorylated product or a triphosphorylated product, and a diphosphorylated product is preferred. Specific examples of the second nucleotide coenzyme include the aforementioned specific examples, and further preferable examples include ADP, GDP, TDP, UDP, CDP, XDP, IDP, dADP, dGDP, dTDP, dUDP, dCDP, dXDP and dIDP.

Combinations of the nucleoside moieties of the first nucleotide coenzyme and the second nucleotide coenzyme in the composition of the present embodiment are not especially limited as long as the nucleoside moieties are different from each other, and examples include combinations of adenosine and inosine, guanosine and adenosine, deoxyadenosine and guanosine, deoxyadenosine and deoxyguanosine, deoxyadenosine and inosine, or inosine and adenosine. Preferably, the nucleoside moiety of the first nucleotide coenzyme is adenosine, and the nucleoside moiety of the second nucleotide coenzyme is inosine.

By using the composition according to the present embodiment, for example, the method of the present embodiment can be performed, and the reaction of the formula (3) can be caused by the cycling method.

By using the composition according to the present embodiment, an amount of change of a signal corresponding to a change of any one of the first nucleotide coenzyme, the conversion product of the first nucleotide coenzyme resulting from the forward reaction, the second nucleotide coenzyme, and the conversion product of the second nucleotide coenzyme resulting from the reverse reaction can be detected. Besides, the change of the signal may be a decreased amount of the first nucleotide coenzyme or the second nucleotide coenzyme, or an increased amount of the conversion product of the first nucleotide coenzyme resulting from the forward reaction or the conversion product of the second nucleotide coenzyme resulting from the reverse reaction. Preferably, the change of the signal is the increased amount of the conversion product of the first nucleotide coenzyme resulting from the forward reaction or the conversion product of the second nucleotide coenzyme resulting from the reverse reaction. As a method for detecting the change, any of known methods such as the HPLC can be employed. In a case where the HPLC is employed, it is performed in the same manner as described with respect to the method of the present embodiment.

By using the composition according to the present embodiment, the amount, contained in a sample, of the kinase forward reaction substrate and/or the phosphorylated product thereof, or the precursor thereof can be calculated. A calculation method is the same as that described with respect to the method of the present embodiment, and can be performed by employing a known method.

The composition according to the present embodiment may optionally contain a detection enzyme able to utilize the conversion product of the first nucleotide coenzyme resulting from the forward reaction but unable to utilize the second nucleotide coenzyme, or a detection enzyme unable to utilize the first nucleotide coenzyme but able to utilize the conversion product of the second nucleotide coenzyme resulting from the reverse reaction. An example of the detection enzyme includes, but is not limited to, adenosine diphosphate-dependent glucokinase (EC 2.7.1.147). In addition, a coenzyme of any one of thio-NADP, thio-NAD, NADP and NAD, glucose and glucose-6-phosphate dehydrogenase are added to the composition of the present embodiment in some cases. By using them, a change of any signal corresponding to the increased amount of the conversion product of the first nucleotide coenzyme resulting from the forward reaction or the conversion product of the second nucleotide coenzyme resulting from the reverse reaction can be detected and measured. Specific description is the same as that made with respect to the method of the present embodiment.

The composition according to the present embodiment is provided as a reagent kit divided into a plurality of parts. For example, a composition containing the second nucleotide coenzyme can be distributed in a reagent kit appropriately divided into two, three or more parts. In this case, a composition containing a detection enzyme able to utilize, for example, thio-NADP, thio-NAD, NADP or NAD, glucose, and the conversion product of the first nucleotide coenzyme resulting from the forward reaction but unable to utilize the second nucleotide coenzyme, or a detection enzyme unable to utilize the first nucleotide coenzyme but able to utilize the conversion product of the second nucleotide coenzyme resulting from the reverse reaction can be distributed in the reagent kit appropriately divided into two, three or more parts. All of these components can be contained dually in some of the divided parts of the reagent kit respectively.

In a case where CK is used, a composition containing ATP, CK and IDP can be appropriately divided into two parts of the reagent kit. Further, NADP, ADP-dependent glucokinase and glucose-6-phosphate dehydrogenase may be appropriately distributed to two divided parts of the reagent kit. All of these components can be contained dually in some of the divided parts of the regent kit respectively.

Now, the present invention will be further specifically described with reference to examples.

### (Confirmation of Nucleotide Specificity of ADP-HK)

It was confirmed that ADP-dependent hexokinase (ADP-HKPII (T-92), Asahi Kasei Pharma Corporation) derived from Pyrococcus furiosus can utilize, as a phosphate donor, merely ADP but cannot utilize IDP and GDP among nucleoside diphosphates. Specifically, glucose produced by an ADP-HKPII reaction is caused to coexist with glucose-6-phosphate dehydrogenase (G6PDH: Toyobo Co., Ltd.) in the presence of NADP, so as to be measured at 37°C as a change of an absorbance at 340 nm based on reduced NADP.

A reaction solution having the following composition was prepared:
50 mmol/L of Tris-HCl pH 8.0
10 mmol/L of glucose
2 mmol/L of magnesium chloride (MgCl₂)
1 mmol/L of NADP
1 u/mL of G6PDH
1 mmol/L of nucleoside diphosphate (ADP, IDP or GDP)

As a reagent blank, one containing saline instead of a nucleoside diphosphate was used.

First, when ADP-HKPII was added to a concentration of 1.25 u/mL in the reaction solution, the change of the absorbance per minute was 110.4, -0.2 and -0.1 (all expressed as absorbance x 1000) respectively in using ADP, IDP and GDP, and thus, the change in the absorbance was hardly observed using IDP and GDP. Next, the enzyme was added in a 80-fold amount, namely, to a concentration of 100 u/mL in the reaction solution. In using ADP, the absorbance was immediately increased beyond 3000 (absorbance x 1000) but in using IDP or GDP, the change of the absorbance caused in nine minutes was respectively 4.8 and 0.7 (absorbance x 1000), and thus, it was confirmed that ADP-HKII substantially does not utilize IDP and GTP as a phosphate donor.

### (Example 1: Confirmation Whether or not Reversible Reaction Proceeds in coexistence of ATP and IDP)

Since it was confirmed that ADP-HKII substantially does not utilize IDP as a hydrogen donor, ATP was selected as a nucleoside triphosphate corresponding to the first nucleotide coenzyme, IDP was selected as a nucleoside diphosphate corresponding to the second nucleotide coenzyme, and it was examined whether or not a CK reaction proceeded in the presence of these.

A reaction solution having the following composition was prepared:
50 mmol/L of PIPES buffer (pH 7.0)
10 mmol/L of MgCl₂
10 mmol/L of glucose
1 mmol/L of ADP
1 mmol/L of IDP
1 mmol/L of NADP
8 mmol/L of N-acetylcysteine (NAC)
1 u/mL of G6PDH
1 u/mL of ADP-HKII

One mL of the reaction solution was heated to 37°C. After adding creatine phosphate thereto to a concentration of 0.05 mmol/L in the reaction solution, CK (derived from rabbit muscle, manufactured by Oriental Yeast Co., Ltd.) was added to a concentration of 10 u/mL, and the resultant was observed to find whether or not an absorbance at 340 nm at 37°C was to change. As a result, it was confirmed that the absorbance based on production of reduced NAD was increased with time. This reveals that both the following reactions were continuously caused to proceed by the CK:

Creatine phosphate + IDP → creatine + ITP

Creatine + ATP → creatine + ADP

### (Example 2: Quantitative Determination of Creatine)

A reaction solution having the following composition was prepared:
50 mmol/L of PIPES buffer (pH 7.0)
10 mmol/L of MgCl₂
10 mmol/L of glucose
2 mmol/L of ATP
1 mmol/L of IDP
1 mmol/L of NADP
8 mmol/L of N-acetylcysteine (NAC)
1 u/mL of G6PDH
1 u/mL of ADP-HKII

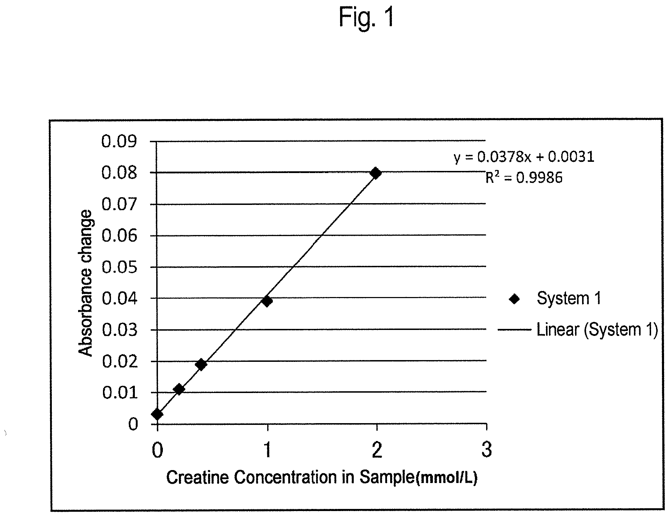

To 1 mL of the reaction solution, CK (manufactured by Asahi Kasei Pharma Corporation, HC-CKII, Catalog No. T-74) was added to a concentration of 100 u/mL. The resultant was preheated at 37°C, and when 0.05 mL of a 0 mmol/L, 0.2 mmol/L, 0.4 mmol/L or 1.2 mmol/L creatine solution was added thereto, an absorbance at 340 nm was increased in proportion to time. Therefore, the absorbance change at 340 nm from minute 3 to minute 5 was recorded. The result is illustrated in Figure 1. As is obvious from Figure 1, the absorbance change was in proportion to the creatine concentration in the sample.

### (Example 3: Rabbit, Combination of Coenzymes)

A reaction solution having the following composition was prepared:
50 mmol/L of PIPES buffer (pH 7.0)
10 mmol/L of MgCl₂
10 mmol/L of glucose
1 mmol/L of NADP
1 mmol/L of nucleoside triphosphate
4 mmol/L of nucleoside diphosphate
8 mmol/L of N-acetylcysteine (NAC)
1 u/mL of G6PDH
1 u/mL of ADP-HKII
100 u/mL of CK (derived from rabbit muscle)

In the reaction solution, as a combination of nucleoside triphosphate (1 mmol/L) and nucleoside diphosphate (4 mmol/L), combinations of dATP/IDP, dATP/GDP, and dATP/dGDP were selected, and with respect to each of the combinations, a difference between an increased amount of the absorbance obtained by adding 0.05 mL of 0.1 mmol/L creatine and an increased amount of the absorbance obtained by adding purified water instead of the creatine was obtained. The absorbance change (absorbance x 10³) caused in five minutes was respectively 34.6 (dATP/IDP), 11.2 (dATP/GDP) and 8.7 (dATP/dGDP), and although there was thus a difference in extent, it was confirmed that the reaction proceeds in employing any of the combinations.

### (Example 4: Measurement of Creatine)

A reaction solution having the following composition was prepared:
50 mmol/L PIPES buffer (pH 7.0)
10 mmol/L of MgCl₂
10 mmol/L of glucose
1 mmol/L of NAD
1 mmol/L of ATP
4 mmol/L of IDP
8 mmol/L of N-acetylcysteine (NAC)
1 u/mL of G6PDH
1 u/mL of ADP-HKII
250 u/mL of CK (derived from rabbit muscle)

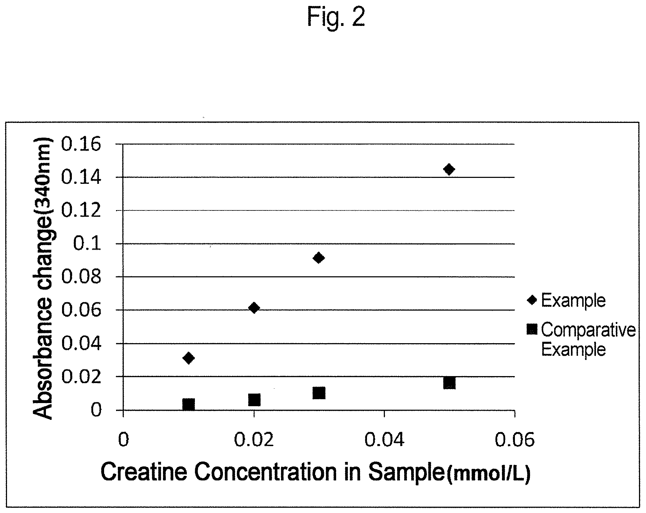

To 1 mL of the reaction solution, 0.05 mL of a 0 mmol/L, 0.01 mmol/L, 0.02 mmol/L, 0.03 mmol/L or 0.05 mmol/L creatine aqueous solution was added, and the resultant was monitored for a change of an absorbance at 340 nm at 37°C. As a comparative example, a solution obtained by removing IDP from the above-described reaction solution was used for performing similar measurement. The absorbance change was measured for five minutes in the example, and the absorbance was measured 10 minutes after starting the reaction in the comparative example, and each of the thus obtained changes was subtracted from that of a reagent blank. The results of the both examples are illustrated in Figure 2. In the present example, the absorbance change was increased about 10 times as much as that of the comparative example.

### (Example 5: Quantitative Determination of Creatinine)

A reaction solution 1 having the following composition was prepared:
50 mmol/L of PIPES-NaOH buffer (pH 7.0)
12 mmol/L of MgCl₂
10 mmol/L of glucose
5.3 mmol/L of IDP
20 u/mL creatinine amidohydrolase (manufactured by Toyobo Co., Ltd.)

In addition, a reaction solution 2 having the following composition was prepared:
200 mmol/L of PIPES buffer (pH 7.0)
2 mmol/L of MgCl₂
4 mmol/L of thio-NAD
4 mmol/L of ATP
10 mmol/L of N-acetylcysteine (NAC)
4 u/mL of G6PDH
4 u/mL of ADP-HKII
1000 u/mL of CK (derived from rabbit muscle)

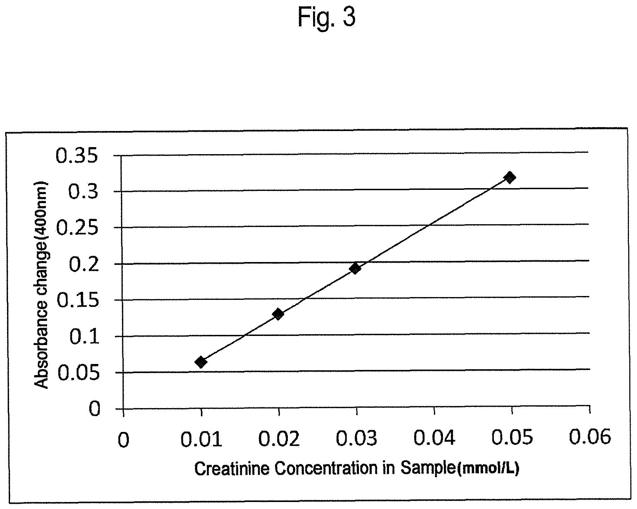

To 0.75 mL of the reaction solution 1, 0.05 mL of a 0.01 mmol/L, 0.02 mmol/L, 0.03 mmol/L or 0.05 mmol/L creatinine solution was added respectively, and the resultant was heated at 37°C for 5 minutes. Thereafter, 0.25 mL of the reaction solution 2 was added thereto, and an absorbance at 400 nm was monitored. An absorbance change caused from minute 2 to minute 7 after the addition of the reaction solution 2 was measured. As a result, as illustrated in Figure 3, the absorbance change was quantitatively increased in accordance with the concentration of creatinine.

### (Example 6 : Quantitative Determination of Creatinine)

A DNA encoding mouse B-type isozyme was obtained by a usual method. This gene was inserted, for cloning, into a host/vector system used for production of CK (manufactured by Asahi Kasei Pharma Corporation, Catalog No. T-74), so as to obtain CK-B (human-derived B-type isozyme) by a usual method.

Besides, a reaction solution 1 having the following composition was prepared:
50 mmol/L of TES buffer (pH 7.5)
12 mmol/L of magnesium chloride
12 mmol/L of glucose
1.5 u/mL of ADP-HK (manufactured by Asahi Kasei Pharma Corporation: T-92)
1.5 u/mL of G6PDH (manufactured by Toyobo Co., Ltd.)
1.5 mmol/L of thio-NAD (manufactured by Oriental Yeast Co., Ltd.)
0.7 mmol/L of ATP

Furthermore, a reaction solution 2 having the following composition was prepared:
50 mmol/L of TES buffer (pH 7.5)
8 mmol/L of IDP
0.5 mmol/L of N-acetylcysteine
0.02% of BSA
16 u/mL of creatinine amidohydrolase (manufactured by Toyobo Co., Ltd.)
200 u/mL of CK-B (human-derived B-type isozyme)

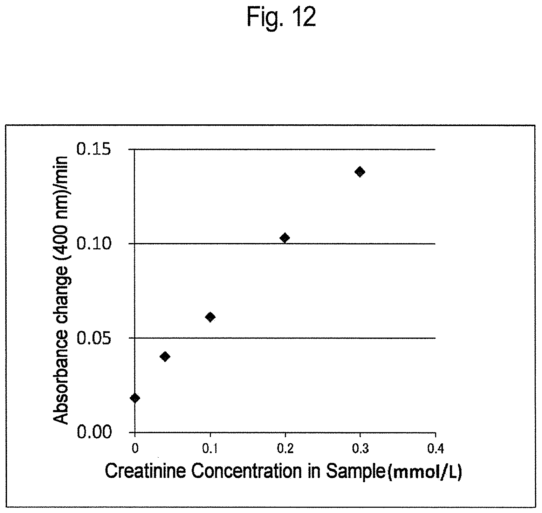

A reaction for converting creatinine to creatine by creatinine amidohydrolase and a CK cycling reaction were simultaneously performed. To 0.75 mL of the reaction solution 1, 0.05 mL of a 0.04 mmol/L, 0.1 mmol/L, 0.2 mmol/L or 0.3 mmol/L creatinine aqueous solution was added, and the resultant was preheated for three minutes at 37°C. Subsequently, 0.25 mL of the reaction solution 2 was added thereto, and the reaction was started. An absorbance change at 400 nm caused minute 3 to minute 4 after the addition of the reaction solution 2 was measured. As a result, as illustrated in Figure 12, the absorbance change was quantitatively increased in accordance with the concentration of the creatinine.

## Claims

1. A measuring method for a kinase forward reaction substrate and/or a phosphorylated product thereof, comprising:
(1) a step of conducting a cycling reaction according to the following formula (1): 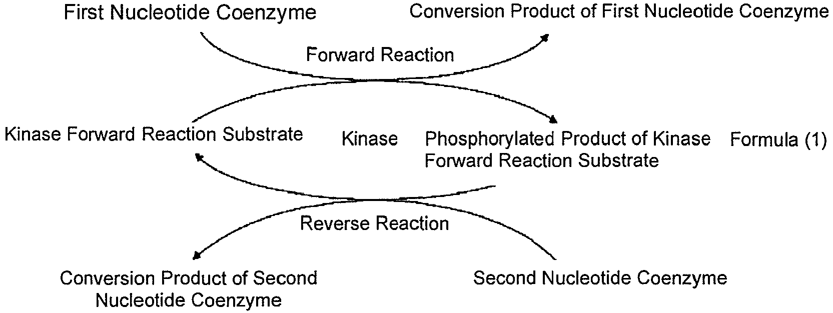 by bringing: (i) a kinase catalyzing a forward reaction for producing a phosphorylated product from a kinase forward reaction substrate and a reverse reaction thereof in the presence of nucleotide coenzymes, the kinase utilizing nucleotide coenzymes at least having different nucleoside moieties in the forward reaction and the reverse reaction, respectively;(ii) a first nucleotide coenzyme of the kinase; and (iii) a second nucleotide coenzyme having a different nucleoside moiety from the first nucleotide coenzyme;
into contact with a sample;
(2) a step of detecting an amount of change of a signal corresponding to a change of at least any one of the first nucleotide coenzyme, a conversion product of the first nucleotide coenzyme, the second nucleotide coenzyme, and a conversion product of the second nucleotide coenzyme; and
(3) a step of calculating, on the basis of the detected change of the signal, an amount the kinase forward reaction substrate and/or the phosphorylated product thereof contained in the sample;
wherein the kinase is creatine kinase; and the forward reaction substrate is creatine.

2. A measuring method for a precursor of a kinase forward reaction substrate, comprising:
(1) a step of conducting a cycling reaction according to the following formula (1): 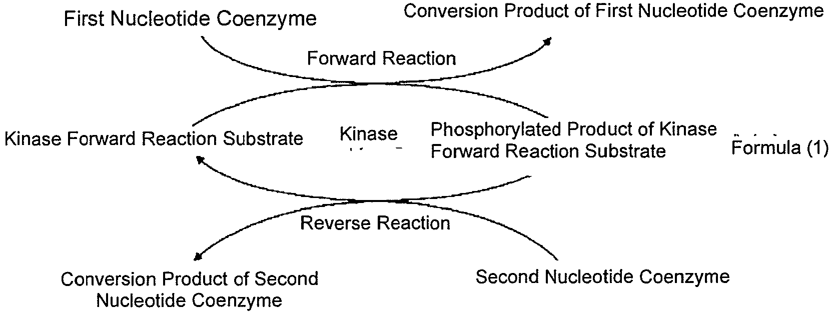 by bringing: (i) a kinase catalyzing a forward reaction for producing a phosphorylated product from a kinase forward reaction substrate and a reverse reaction thereof in the presence of nucleotide coenzymes, the kinase utilizing nucleotide coenzymes at least having different nucleoside moieties in the forward reaction and the reverse reaction, respectively;(ii) a first nucleotide coenzyme of the kinase; and (iii) a second nucleotide coenzyme having a different nucleoside moiety from the first nucleotide coenzyme;
into contact with a sample having been subjected to a quantitative derivation treatment from the precursor to the kinase forward reaction substrate and/or the phosphorylated product thereof;
(2) a detection step according to step (2) of claim 1; and
(3) a step of calculating, on the basis of the detected change of the signal, an amount of the precursor contained in the sample;
wherein the kinase is creatine kinase; the forward reaction substrate is creatine; and the precursor is creatinine.

3. The measuring method according to claim 2, wherein creatinine is brought into contact with creatinine amidohydrolase (EC 3.5.2.10) in the presence of water in the quantitative derivation treatment from the precursor to the kinase forward reaction substrate and/or the phosphorylated product thereof.

4. The measuring method according to any one of claims 1 to 3, wherein the nucleoside moiety of the first nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.

5. The measuring method according to any one of claims 1 to 4, wherein the nucleoside moiety of the second nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.

6. The measuring method according to any one of claims 1 to 5, wherein a combination of the nucleoside moiety of the first nucleotide coenzyme and the nucleoside moiety of the second nucleotide coenzyme is a combination of adenosine and inosine, guanosine and adenosine, deoxyadenosine and guanosine, deoxyadenosine and deoxyguanosine, deoxyadenosine and inosine, or inosine and adenosine.

7. The measuring method according to any one of claims 1 to 6, wherein the nucleoside moiety of the first nucleotide coenzyme is adenosine, and the nucleoside moiety of the second nucleotide coenzyme is inosine.

8. The measuring method according to any one of claims 1 to 7, wherein the first nucleotide coenzyme is adenosine triphosphate (ATP), and the second nucleotide coenzyme is inosine diphosphate (IDP).

9. The measuring method according to any one of claims 1 to 8, wherein in the step of detecting the change of the signal, an amount of change of a signal corresponding to an increased amount of the conversion product of the first nucleotide coenzyme resulting from the forward reaction or the conversion product of the second nucleotide coenzyme resulting from the reverse reaction is detected by using a detection enzyme able to utilize the conversion product of the first nucleotide coenzyme resulting from the forward reaction but unable to utilize the second nucleotide coenzyme, or a detection enzyme unable to utilize the first nucleotide coenzyme but able to utilize the conversion product of the second nucleotide coenzyme resulting from the reverse reaction.

10. The measuring method according to any one of claims 1 to 9, wherein in the step of detecting the change of the signal, an amount of change of a signal varied in accordance with an increased amount of adenosine diphosphate (ADP) is detected by using adenosine diphosphate (ADP)-dependent glucokinase (EC 2.7.1.147) in the presence of glucose.

11. The measuring method according to any one of claims 1 to 10, wherein in the step of detecting the amount of change of the signal, an amount of change of a signal varied in accordance with an increased amount of adenosine diphosphate (ADP) is detected by using adenosine diphosphate (ADP)-dependent glucokinase (EC 2.7.1.147) in the presence of any one of coenzymes of thionicotinamide adenine dinucleotide phosphate (thio-NADP), thionicotinamide adenine dinucleotide (thio-NAD), nicotinamide adenine dinucleotide phosphate (NADP) and nicotinamide adenine dinucleotide (NAD), glucose, and glucose 6-phosphate dehydrogenase.

12. A reagent kit for measuring a kinase forward reaction substrate and/or a phosphorylated product thereof, comprising:
(a) a kinase that catalyzes a forward reaction for producing a phosphorylated product from the kinase forward reaction substrate and a reverse reaction thereof in the presence of nucleotide coenzymes, and utilizes nucleotide coenzymes at least having different nucleoside moieties in the forward reaction and the reverse reaction, respectively,
(b) a first nucleotide coenzyme for the forward reaction; and
(c) a second nucleotide coenzyme for the reverse reaction having a different nucleoside moiety from the first nucleotide coenzyme,
wherein the kinase is creatine kinase; and the kinase forward reaction substrate is creatine.

13. A reagent kit for measuring a precursor of a kinase forward reaction substrate, and to be used after the precursor is subjected to a quantitative derivation treatment from the precursor to the kinase forward reaction substrate and/or the phosphorylated product thereof, comprising components (a), (b) and (c) of claim 12;
wherein the kinase is creatine kinase; the kinase forward reaction substrate is creatine; and the precursor is creatinine; and
wherein creatine is to be derived by bringing creatinine into contact with creatinine amidohydrolase (EC 3.5.2.10) in the presence of water in the quantitative derivation treatment from the precursor to the kinase forward reaction substrate and/or the phosphorylated product thereof.

14. The reagent kit according to claim 12 or 13, wherein the nucleoside moiety of the first nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.

15. The reagent kit according to any one of claims 12 to 14, wherein the nucleoside moiety of the second nucleotide coenzyme is any one of adenosine, guanosine, thymidine, uridine, cytidine, xanthosine, inosine, deoxyadenosine, deoxyguanosine, deoxythymidine, deoxyuridine, deoxycytidine, deoxyxanthosine and deoxyinosine.

16. The reagent kit according to any one of claims 12 to 15, wherein a combination of the nucleoside moiety of the first nucleotide coenzyme and the nucleoside moiety of the second nucleotide coenzyme is a combination of adenosine and inosine, guanosine and adenosine, deoxyadenosine and guanosine, deoxyadenosine and deoxyguanosine, deoxyadenosine and inosine, or inosine and adenosine.

17. The reagent kit according to any one of claims 12 to 16, wherein the nucleoside moiety of the first nucleotide coenzyme is adenosine, and the nucleoside moiety of the second nucleotide coenzyme is inosine.

18. The reagent kit according to any one of claims 12 to 17, wherein the first nucleotide coenzyme is adenosine triphosphate (ATP), and the second nucleotide coenzyme is inosine diphosphate (IDP).

19. The reagent kit according to any one of claims 12 to 17, further comprising a detection enzyme able to utilize a conversion product of the first nucleotide coenzyme resulting from the forward reaction but unable to utilize the second nucleotide coenzyme, or a detection enzyme unable to utilize the first nucleotide coenzyme but able to utilize a conversion product of the second nucleotide coenzyme resulting from the reverse reaction.

20. The reagent kit according to claim 19, wherein the detection enzyme is adenosine diphosphate (ADP)-dependent glucokinase (EC 2.7.1.147), and the composition further comprises glucose.

21. The reagent kit according to claim 19, further comprising thionicotinamide adenine dinucleotide phosphate (thio-NADP), thionicotinamide adenine dinucleotide (thio-NAD), nicotinamide adenine dinucleotide phosphate (NADP) or nicotinamide adenine dinucleotide (NAD); and glucose-6-phosphate dehydrogenase.

22. The reagent kit according to claim 13 or 14,
wherein the measurement target is creatinine as the precursor,
the reagent kit is to be used after a quantitative derivation treatment from the creatinine to creatine by bringing the creatinine into contact with creatinine amidohydrolase (EC 3.5.2.10) in the presence of water, and comprises:
(a) creatine kinase;
(b) adenosine triphosphate (ATP);
(c) inosine diphosphate (IDP);
(d) adenosine diphosphate (ADP)-dependent glucokinase (EC 2.7.1.147) ;
(e) glucose;
(f) thionicotinamide adenine dinucleotide phosphate (thio-NADP), thionicotinamide adenine dinucleotide (thio-NAD), nicotinamide adenine dinucleotide phosphate (NADP) or nicotinamide adenine dinucleotide (NAD); and
(g) glucose-6-phosphate dehydrogenase.

23. A composition comprising:
(a) a kinase that catalyzes a forward reaction for producing a phosphorylated product from a kinase forward reaction substrate and a reverse reaction thereof in the presence of nucleotide coenzymes, and utilizes nucleotide coenzymes at least having different nucleoside moieties in the forward reaction and the reverse reaction, respectively,
(b) a first nucleotide coenzyme for the forward reaction; and
(c) a second nucleotide coenzyme for the reverse reaction having a different nucleoside moiety from the first nucleotide coenzyme,
wherein the kinase is creatine kinase; and the kinase forward reaction substrate is creatine.

## Patentansprüche

1. Messverfahren für ein Substrat einer Kinase-Hinreaktion und/oder ein phosphoryliertes Produkt davon, umfassend:
(1) einen Schritt zum Durchführen einer zyklischen Reaktion gemäß der folgenden Formel (1): 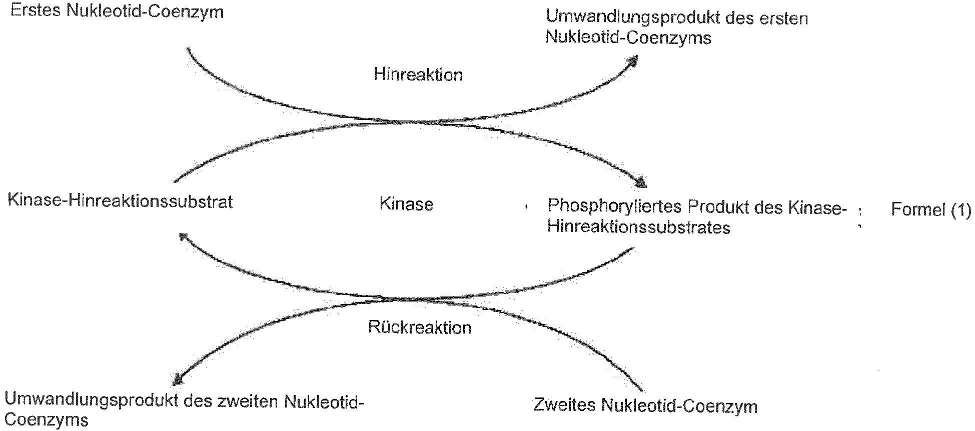 durch Bringen von: (i) einer Kinase, die eine Hinreaktion zur Herstellung eines phosphorylierten Produkts eines Substrats einer Kinase-Hinreaktion und eine Rückreaktion davon in Gegenwart von Nukleotid-Coenzymen katalysiert, wobei die Kinase Nukleotid-Coenzyme verwendet, die zumindest unterschiedliche Nukleosideinheiten in der Hinreaktion bzw. die Rückreaktion aufweisen; (ii) eines ersten Nukleotid-Coenzyms der Kinase; und (iii) eines zweiten Nukleotid-Coenzyms mit einer anderen Nukleosid-Einheit als das erste Nukleotid-Coenzym;
in Kontakt mit einer Probe;
(2) einen Schritt zum Erfassen des Betrags einer Änderung eines Signals, das einer Änderung von mindestens einem von dem ersten Nukleotid-Coenzym, einem Umwandlungsprodukt des ersten Nukleotid-Coenzyms, dem zweiten Nukleotid-Coenzym und einem Umwandlungsprodukt des zweiten Nukleotid-Coenzym entspricht; und
(3) einen Schritt des Berechnens einer Menge des Substrats der Kinase-Hinreaktion und/oder des phosphorylierten Produkts davon, das in der Probe enthalten ist, auf der Basis der erfassten Änderung des Signals;
worin die Kinase Kreatinkinase ist; und das Substrat der Hinreaktion Kreatin ist.

2. Messverfahren für eine Vorstufe eines Substrats einer Kinase Hinreaktion, umfassend:
(1) einen Schritt zum Durchführen einer zyklischen Reaktion gemäß der folgenden Formel (1): 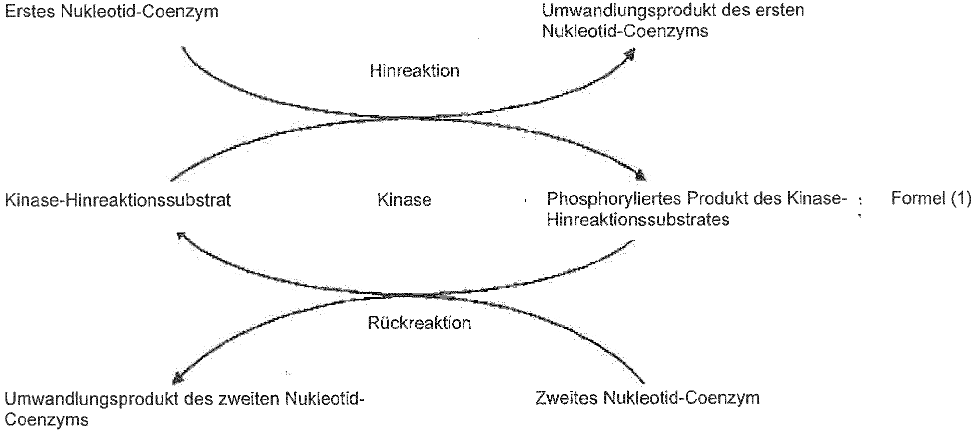 durch Bringen von: (i) einer Kinase, die eine Hinreaktion zur Herstellung eines phosphorylierten Produkts eines Substrats einer Kinase-Hinreaktion und eine Rückreaktion davon in Gegenwart von Nukleotid-Coenzymen katalysiert, wobei die Kinase Nukleotid-Coenzyme verwendet, die zumindest unterschiedliche Nukleosideinheiten in der Hinreaktion bzw. der Rückreaktion aufweisen: (ii) eines ersten Nukleotid-Coenzyms der Kinase; und (iii) eines zweiten Nukleotid-Coenzyms mit einer anderen Nukleosid-Einheit als das erste Nukleotid-Coenzym;
in Kontakt mit einer Probe, die einer quantitativen Derivatisierungsbehandlung von der Vorstufe zu dem Substrat der Kinase-Hinreaktion und/oder dem phosphorylierten Produkt davon unterzogen wurde;
(2) ein Erfassungsschritt gemäß Schritt (2) nach Anspruch 1; und
(3) einen Schritt des Berechnens einer Menge der in der Probe enthaltenen Vorstufe auf der Basis der erfassten Änderung des Signals;
worin die Kinase Kreatinkinase ist; das Substrat der Hinreaktion Kreatin ist; und die Vorstufe Kreatinin ist.

3. Messverfahren nach Anspruch 2, wobei Kreatinin in Kontakt mit Kreatininamidohydrolase (EC 3.5.2.10) in Gegenwart von Wasser bei der quantitativen Derivatisierungsbehandlung von der Vorstufe zu dem Substrat der Kinase-Hinreaktion und/oder dem phosphorylierten Produkt davon gebracht wird.

4. Messverfahren nach einem der Ansprüche 1 bis 3, wobei die Nukleosid-Einheit des ersten Nukleotid-Coenzyms eines von Adenosin, Guanosin, Thymidin, Uridin, Cytidin, Xanthosin, Inosin, Deoxyadenosin, Deoxyguanosin, Deoxythymidin, Deoxyuridin, Deoxycytidin, Deoxyxanthosin und Deoxyinosin ist.

5. Messverfahren nach einem der Ansprüche 1 bis 4, wobei die Nukleosid-Einheit des zweiten Nukleotid-Coenzyms eines von Adenosin, Guanosin, Thymidin, Uridin, Cytidin, Xanthosin, Inosin, Deoxyadenosin, Deoxyguanosin, Deoxythymidin, Deoxyuridin, Deoxycytidin, Deoxyxanthosin und Deoxyinosin ist.

6. Messverfahren nach einem der Ansprüche 1 bis 5, wobei eine Kombination der Nukleosid-Einheit des ersten Nukleotid-Coenzyms und der Nukleosid-Einheit des zweiten Nukleotid-Coenzyms eine Kombination von Adenosin und Inosin, Guanosin und Adenosin, Deoxyadenosin und Guanosin, Deoxyadenosin und Deoxyguanosin, Deoxyadenosin und Inosin oder Inosin und Adenosin ist.

7. Messverfahren nach einem der Ansprüche 1 bis 6, wobei die Nukleosid-Einheit des ersten Nukleotid-Coenzyms Adenosin ist, und die Nukleosid-Einheit des zweiten Nukleotid-Coenzyms Inosin ist.

8. Messverfahren nach einem der Ansprüche 1 bis 7, wobei das erste Nukleotid-Coenzym Adenosintriphosphat (ATP) und das zweite Nukleotid-Coenzym Inosindiphosphat (IDP) ist.

9. Messverfahren nach einem der Ansprüche 1 bis 8, wobei in dem Schritt des Erfassens der Änderung des Signals ein Betrag einer Änderung eines Signals, der einer erhöhten Menge des Umwandlungsprodukts des ersten Nukleotid-Coenzyms entspricht, das sich aus der Hinreaktion ergibt oder des Umwandlungsprodukts des zweiten Nukleotid-Coenzyms, das sich aus der Rückreaktion ergibt, unter Verwendung eines Detektionsenzyms nachgewiesen wird, das das aus der Hinreaktion resultierende Umwandlungsprodukt des ersten Nukleotid-Coenzyms nutzen kann, aber das zweite Nukleotid-Coenzym nicht nutzen kann, oder eines Detektionsenzyms, das das erste Nukleotid-Coenzym nicht nutzen kann, aber das aus der Rückreaktion resultierende Umwandlungsprodukt des zweiten Nukleotid-Coenzyms nutzen kann.

10. Messverfahren nach einem der Ansprüche 1 bis 9, wobei in dem Schritt des Erfassens der Änderung des Signals ein Betrag der Änderung eines Signals, der gemäß einer erhöhten Menge an Adenosindiphosphat (ADP) variiert wird, unter Verwendung von Adenosindiphosphat (ADP) -abhängiger Glucokinase (EC 2.7.1.147) in Gegenwart von Glucose erfasst wird.

11. Messverfahren nach einem der Ansprüche 1 bis 10, wobei in dem Schritt des Erfassens des Betrags der Änderung des Signals ein Betrag der Änderung eines Signals, der gemäß einer erhöhten Menge an Adenosindiphosphat (ADP) variiert wird, unter Verwendung von Adenosindiphosphat (ADP) -abhängiger Glucokinase (EC 2.7.1.147) in Gegenwart eines der Coenzyme von Thionicotinamidadenindinukleotidphosphat (Thio-NADP), Thionicotinamidadenindinukleotid (Thio-NAD), Nikotinamidadenindinukleotidphosphat (NADP) und Nicotinamidadenindinukleotid (NAD), Glucose und Glucose-6-phosphat-Dehydrogenase erfasst wird.

12. Reagenzienkit zum Messen eines Substrats einer Kinase-Hinreaktion und/oder eines phosphorylierten Produkts davon, umfassend:
(a) eine Kinase, die eine Hinreaktion zur Herstellung eines phosphorylierten Produkts von dem Substrat einer Kinase-Hinreaktion und eine Rückreaktion davon in Gegenwart von Nukleotid-Coenzymen katalysiert, und Nukleotid-Coenzyme nutzt, die zumindest jeweils unterschiedliche Nukleosid-Einheiten bei der Hinreaktion bzw. der Rückreaktion aufweisen;
(b) ein erstes Nukleotid-Coenzym für die Hinreaktion; und
(c) ein zweites Nukleotid-Coenzym für die Rückreaktion, das eine andere Nukleosid-Einheit als das erste Nukleotid-Coenzym aufweist,
wobei die Kinase Kreatinkinase ist; und das Substrat der Kinase-Hinreaktion Kreatin ist.

13. Reagenzienkit zum Messen einer Vorstufe eines Substrats einer Kinase-Hinreaktion, und zur Verwendung, nachdem die Vorstufe einer quantitativen Derivatisierungsbehandlung von der Vorstufe zu dem Substrat der Kinase-Hinreaktion und/oder dem phosphorylierten Produkt davon unterzogen wurde, umfassend die Komponenten (a), (b) und (c) von Anspruch 12;
wobei die Kinase Kreatinkinase ist; das Substrat der Kinase-Hinreaktion Kreatin ist; und die Vorstufe Kreatinin ist; und
wobei Kreatin dadurch derivatisiert wird, dass Kreatinin mit Kreatininamidohydrolase (EC 3.5.2.10) in Gegenwart von Wasser bei der quantitativen Derivatisierungsbehandlung von der Vorstufe zu dem Substrat der Kinase-Hinreaktion und/oder dem phosphorylierten Produkt davon in Kontakt gebracht wird.

14. Reagenzienkit nach Anspruch 12 oder 13, wobei die Nukleosid-Einheit des ersten Nukleotid-Coenzyms eine von Adenosin, Guanosin, Thymidin, Uridin, Cytidin, Xanthosin, Inosin, Deoxyadenosin, Deoxyguanosin, Deoxythymidin, Deoxyuridin, Deoxycytidin, Deoxyxanthosin und Deoxyinosin ist.

15. Reagenzienkit nach einem der Ansprüche 12 bis 14 , wobei die Nukleosid-Einheit des zweiten Nukleotid-Coenzyms eine von Adenosin, Guanosin, Thymidin, Uridin, Cytidin, Xanthosin, Inosin, Deoxyadenosin, Deoxyguanosin, Deoxythymidin, Deoxyuridin, Deoxycytidin, Deoxyxanthosin und Deoxyinosin ist.

16. Reagenzienkit nach einem der Ansprüche 12 bis 15 , wobei eine Kombination der Nukleosid-Einheit des ersten Nukleotid-Coenzyms und die Nukleosid-Einheit des zweiten Nukleotid-Coenzyms eine Kombination von Adenosin und Inosin, Guanosin und Adenosin, Deoxyadenosin und Guanosin, Deoxyadenosin und Deoxyguanosin, Deoxyadenosin und Inosin oder Inosin und Adenosin ist.

17. Reagenzienkit nach einem der Ansprüche 12 bis 16 , wobei die Nukleosid-Einheit des ersten Nukleotid-Coenzyms Adenosin ist, und die Nukleosid-Einheit des zweiten Nukleotid-Coenzyms Inosin ist.

18. Reagenzienkit nach einem der Ansprüche 12 bis 17 , wobei das erste Nukleotid-Coenzym Adenosintriphosphat (ATP) ist, und die zweite Nukleotid-Coenzym Inosindiphosphat (IDP) ist.

19. Reagenzienkit nach einem der Ansprüche 12 bis 17, ferner umfassend ein Detektions-Enzym, das in der Lage ist, ein Umwandlungspodukt des ersten ersten Nukleotid-Coenzyms zu verwenden, das aus der Hinreaktion resultiert, aber nicht in der Lage ist, das zweite Nukleotid-Coenzym zu verwenden, oder ein Detektions-Enzym zu verwenden, das nicht in der Lage ist, das erste Nukleotid-Coenzym zu verwenden, aber in der Lage ist, ein Umwandlungsprodukt des zweiten Nukleotid-Coenzyms zu verwenden, das aus der Rückreaktion resultiert.

20. Reagenzienkit nach Anspruch 19, wobei das Detektions-Enzym Adenosindiphosphat (ADP) -abhängige Glukokinase (EC 2.7.1.147) ist, und die Zusammensetzung weiter Glucose umfasst.

21. Reagenzienkit nach Anspruch 19n, ferner umfassend Thionicotinamidadenindinukleotidphosphat (Thio-NADP), Thionicotinamidadenindinukleotid (Thio-NAD), Nicotinamidadenindinukleotidphosphat (NADP) oder Nicotinamidadenindinukleotid (NAD); und Glucose-6-phosphat-Dehydrogenase.

22. Reagenzienkit nach Anspruch 13 oder 14,
wobei das Messziel Kreatinin als Vorstufe ist,
das Reagenzienkit nach einer quantitativen Derivatisierungsbehandlung von dem Kreatinin zu Kreatin genutzt werden soll, wobei Kreatinin mit Kreatininamidohydrolase (EC 3.5.2.10) in Gegenwart von Wasser in Kontakt gebracht wird, und umfasst:
(a) Kreatinkinase;
(b) Adenosintriphosphat (ATP);
(c) Inosindiphosphat (IDP);
(d) Adenosindiphosphat (ADP) -abhängige Glucokinase (EC 2.7.1.147);
(e) Glucose;
(f) Thionicotinamidadenindinukleotidphosphat (Thio-NADP), Thionicotinamidadenindinukleotid (Thio-NAD), Nicotinamidadenindinukleotidphosphat (NADP) oder Nicotinamidadenindinukleotid (NAD); und
(g) Glucose-6-phosphat-Dehydrogenase.

23. Zusammensetzung, umfassend:
(a) eine Kinase, die eine Hinreaktion zur Herstellung eines phosphorylierten Produkts aus einem Substrat einer Kinase-Hinreaktion und einer Rückreaktion davon in Gegenwart von Nukleotid-Coenzymen katalysiert, und Nukleotid-Coenzyme verwendet, die mindestens verschiedene Nukleosid-Einheiten in der Hinreaktion bzw. der Rückreaktion aufweisen,
(b) ein erstes Nukleotid-Coenzym für die Hinreaktion; und
(c) ein zweites Nukleotid-Coenzym für die Rückreaktion mit einer anderen Nukleosideinheit als das erste Nukleotid-Coenzym,
worin die Kinase Kreatinkinase ist; und das Substrat der Kinase-Hinreaktion Kreatin ist.

## Revendications

1. Procédé de mesure d'un substrat de réaction directe de kinase et/ou d'un produit phosphorylé de celui-ci, comprenant:
(1) une étape de réalisation d'une réaction de cyclage selon la formule (1) suivante: 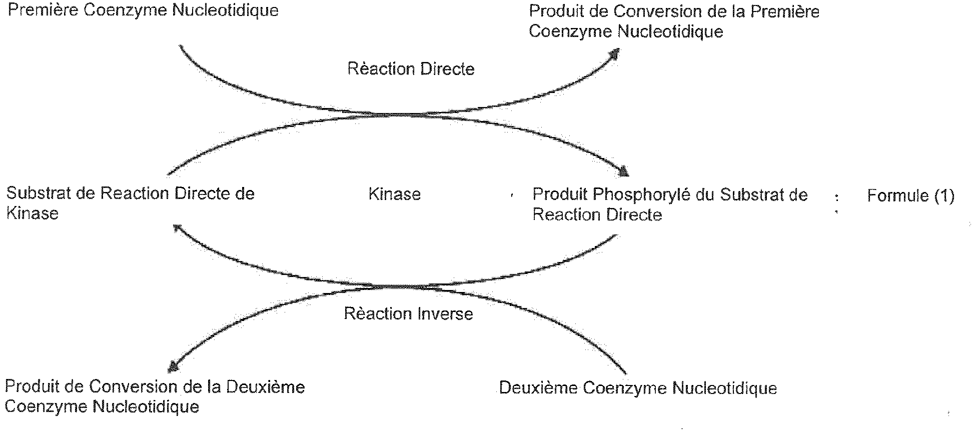 par mise en contact de: (i) une kinase catalysant une réaction directe pour produire un produit phosphorylé à partir d'un substrat de réaction directe de kinase et une réaction inverse de celle-ci en présence des coenzymes nucléotidiques, la kinase utilisant des coenzymes nucléotidiques ayant au moins des fragments nucléosidiques différentes dans la réaction directe et la réaction inverse, respectivement: (ii) une premiere coenzyme nucléotidique de la kinase; et (iii) une deuxième coenzyme nucléotidique ayant un fragment nucléosidique différent de la première coenzyme nucléotidique; avec un échantillon;
(2) une étape de détection d'une quantité de changement d'un signal correspondant à un changement d'au moins l'un quelconque de la première coenzyme nucléotidique, d'un produit de conversion de la première coenzyme nucléotidique, de la deuxième coenzyme nucléotidique, et d'un produit de conversion de la deuxième coenzyme nucléotidique; et
(3) une étape de calcul, sur la base du changement détecté du signal, d'une quantité du substrat de réaction directe de kinase et/ou de son produit phosphorylé contenu dans l'échantillon;
dans lequel la kinase est la créatine kinase; et le substrat de la réaction directe est la créatine.

2. Procédé de mesure d'un précurseur d'un substrat de réaction directe de kinase, comprenant:
(1) une étape de réalisation d'une réaction de cyclage selon la formule (1) suivante: 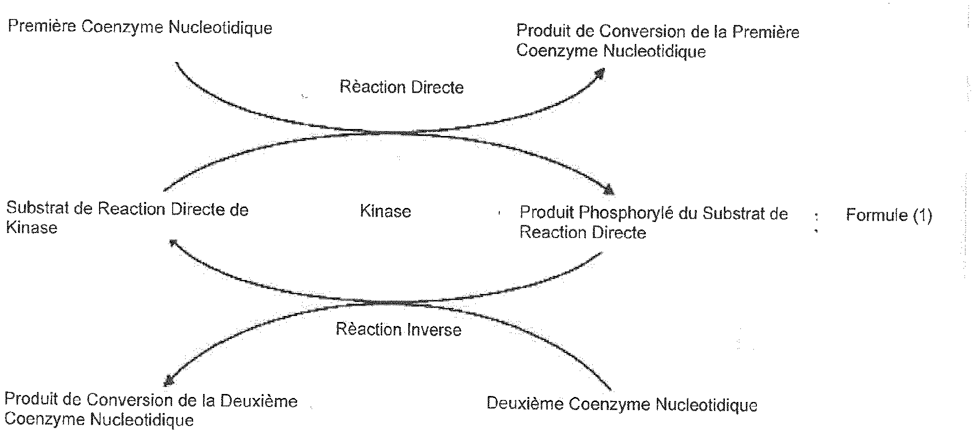 par mise en contact de: (i) une kinase catalysant une réaction directe pour produire un produit phosphorylé à partir d'un substrat de réaction directe de kinase et une réaction inverse de celle-ci en présence des coenzymes nucléotidiques, la kinase utilisant des coenzymes nucléotidiques ayant au moins des parties nucléosidiques différentes dans la réaction directe et la réaction inverse, respectivement: (ii) une premiere coenzyme nucléotidique de la kinase; et (iii) une deuxième coenzyme nucléotidique ayant un fragment nucléosidique différent de la première coenzyme nucléotidique;
avec un échantillon qui a été soumis à un traitement de dérivation quantitative a partir du précurseur au substrat de réaction directe de kinase et/ou de son produit phosphorylé;
(2) une étape de détection selon l'étape (2) de la revendication 1; et
(3) une étape de calcul, sur la base du changement détecté du signal, d'une quantité du précurseur contenu dans l'échantillon;
dans lequel la kinase est la créatine kinase; le substrat de la réaction directe est la créatine; et le précurseur est la créatinine.

3. Procédé de mesure selon la revendication 2, dans lequel la créatinine est mis en contact avec la créatinine amidohydrolase (EC 3.5.2.10) en présence d'eau dans le traitement de dérivation quantitative à partir du précurseur du substrat de réaction directe de kinase et/ou de son produit phosphorylé.

4. Procédé de mesure selon l'une quelconque des revendications 1 à 3, dans lequel le fragment nucléosidique de la première coenzyme nucléotidique est l'une quelconque parmi l'adénosine, la guanosine, la thymidine, l'uridine, la cytidine, la xanthosine, l'inosine, la désoxyadénosine, la désoxyguanosine, la désoxythymidine, la désoxyuridine, la désoxycytidine, la désoxyxanthosine et la désoxyinosine.

5. Procédé de mesure selon l'une quelconque des revendications 1 à 4, dans lequel le fragment nucléosidique de la deuxième coenzyme nucléotidique est l'une quelconque parmi l'adénosine, la guanosine, la thymidine, l'uridine, la cytidine, la xanthosine, l'inosine, la désoxyadénosine, la désoxyguanosine, la désoxythymidine, la désoxyuridine, la désoxycytidine, la désoxyxanthosine et la désoxyinosine.

6. Procédé de mesure selon l'une quelconque des revendications 1 à 5, dans lequel une combinaison du fragment nucléosidique de la première coenzyme nucléotidique et du fragment nucléosidique de la deuxième coenzyme nucléotidique est une combinaison d'adénosine et d'inosine, de guanosine et d'adénosine, de désoxyadénosine et de guanosine, de désoxyadénosine et de désoxyguanosine, de désoxyadénosine et d'inosine, ou d'inosine et d'adénosine.

7. Procédé de mesure selon l'une quelconque des revendications 1 à 6, dans lequel le fragment nucléosidique de la première coenzyme nucléotidique est l'adénosine, et le fragment nucléosidique de la deuxième coenzyme nucléotidique est l'inosine.

8. Procédé de mesure selon l'une quelconque des revendications 1 à 7, dans lequel la première coenzyme nucléotidique est l'adénosine triphosphate (ATP), et la deuxième coenzyme nucléotidique est l'inosine diphosphate (IDP).

9. Procédé de mesure selon l'une quelconque des revendications 1 à 8, dans lequel, dans l'étape de détection du changement du signal, une quantité de changement d' un signal correspondant à une quantité accrue du produit de conversion de la première coenzyme nucléotidique résultant de la réaction directe ou du produit de conversion de la deuxième coenzyme nucléotidique résultant de la réaction inverse est détectée en utilisant une enzyme de détection capable d' utiliser le produit de conversion de la première coenzyme nucléotidique résultant de la réaction directe mais incapable d' utiliser la deuxième coenzyme nucléotidique, ou un enzyme de détection incapable d'utiliser la première coenzyme nucléotidique mais capable d'utiliser le produit de conversion de la deuxième coenzyme nucléotidique résultant de la réaction inverse.

10. Procédé de mesure selon l'une quelconque des revendications 1 à 9, dans lequel dans l'étape de détection du changement du signal, une quantité de changement d'un signal varié en fonction d'une quantité accrue d'adénosine diphosphate (ADP) est détectée en utilisant la glucokinase dépendante de l'adénosine diphosphate (ADP) (EC 2.7.1.147) en présence de glucose.

11. Procédé de mesure selon l'une quelconque des revendications 1 à 10, dans lequel, à l'étape de détection de la quantité de changement du signal, une quantité de changement d'un signal varié en fonction d'une quantité accrue d'adénosine diphosphate (ADP) est détectée en utilisant une glucokinase dépendante de l'adénosine diphosphate (ADP) (EC 2.7.1.147) en présence de l'un des coenzymes de thionicotinamide adénine dinucléotide phosphate (thio -NADP), thionicotinamide adénine dinucléotide (thio-NAD), nicotinamide adénine dinucléotide phosphate (NADP) et nicotinamide adénine dinucléotide (NAD), glucose et glucose 6-phosphate déshydrogénase.

12. Un kit de réactifs pour mesurer un substrat de réaction direct de kinase et/ou un produit phosphorylé de celui - ci, comprenant:
(a) une kinase qui catalyse une réaction directe pour produire un produit phosphorylé à partir du substrat de réaction directe de kinase et une réaction inverse de celle - ci en présence des coenzymes nucléotidiques, et utilise les coenzymes nucléotides ayant au moins des fragments differents de nucleoside dans la réaction directe et la réaction inverse, respectivement,
(b) une premiere coenzyme nucléotidique pour la réaction directe; et
(c) une deuxième coenzyme nucléotidique pour la réaction inverse ayant un fragment nucléosidique différent de la première coenzyme nucléotidique,
dans lequel la kinase est la créatine kinase; et le substrat de la réaction directe de la kinase est la créatine.

13. Un kit de réactifs pour mesurer un précurseur d'un substrat de réaction directe d'une kinase, et pour être utilisé après que le précurseur est soumis à un traitement de dérivation quantitative à partir du précurseur du substrat de la réaction directe de la kinase et/ou le produit phosphorylé de celle - ci, comprenant les composants (a), (b) et (c) selon la revendication 12;
dans lequel la kinase est la créatine kinase; le substrat de la réaction directe de la kinase est la créatine; et le précurseur est la créatinine; et
dans lequel la créatine doit être dérivé en mettant la créatinine en contact avec la créatinine amidohydrolase (EC 3.5.2.10) en présence d'eau dans le traitement de dérivation quantitative à partir du précurseur du substrat de la réaction directe de la kinase et/ou du produit phosphorylé de celle - ci.

14. Le kit de réactifs selon la revendication 12 ou 13, dans lequel le fragment de nucléoside de la première coenzyme nucléotidique est l'une quelconque de l'adénosine, la guanosine, la thymidine, l'uridine, la cytidine, la xanthosine, l'inosine, la désoxyadénosine, désoxyguanosine, désoxythymidine, la désoxyuridine, la désoxycytidine, la désoxyxanthosine et la désoxyinosine.

15. Le kit de réactif selon l'une quelconque des revendications 12 à 14, dans lequel le fragment de nucléoside de la deuxième coenzyme nucléotidique est l'une quelconque de l'adénosine, la guanosine, la thymidine, l'uridine, la cytidine, la xanthosine, l'inosine, la désoxyadénosine, la désoxyguanosine, la désoxythymidine, la désoxyuridine, la désoxycytidine, la désoxyxanthosine et la désoxyinosine.

16. Le kit de réactif selon l'une quelconque des revendications 12 à 15, dans lequel une combinaison du fragment de nucléoside de la première coenzyme nucléotidique et du fragment de nucléoside de la deuxième coenzyme nucléotidique est une combinaison de l'adénosine et de l'inosine, de la guanosine et de l'adénosine, de la désoxyadénosine et de la guanosine, de la désoxyadénosine et de la désoxyguanosine, de la désoxyadénosine et de l'inosine, ou de l'inosine et de l'adénosine.

17. Le kit de réactif selon l'une quelconque des revendications 12 à 16, dans lequel le fragment du nucléoside de la première coenzyme nucléotidique est l'adénosine, et le fragment du nucléoside de la deuxième coenzyme nucléotidique est l'inosine.

18. Le kit de réactif selon l'une quelconque des revendications 12 à 17, dans lequel la première coenzyme nucléotidique est l'adénosine triphosphate (ATP), et la deuxième coenzyme nucléotidique est l'inosine diphosphate (IDP).

19. Le kit de réactif selon l'une quelconque des revendications 12 à 17, comprenant en outre une enzyme de détection capable d' utiliser un produit de conversion de la première coenzyme nucléotidique résultant de la réaction directe, mais incapable d'utiliser la deuxieme coenzyme nucléotidique, ou une enzyme de détection incapable d'utiliser la première coenzyme nucléotidique mais capable d'utiliser un produit de conversion de la deuxième coenzyme nucléotidique résultant de la réaction inverse.

20. Le kit de réactif selon la revendication 19, dans lequel l'enzyme de détection est la glucokinase dépendante de l'adénosine diphosphate (ADP) (EC 2.7.1.147), et la composition comprend en outre glucose.

21. Le kit de réactif selon la revendication 19, comprenant en outre le thionicotinamide phosphate adénine dinucléotide (thio-NADP), thionicotinamide adénine dinucléotide (thio-NAD), le nicotinamide adénine dinucléotide phosphate (NADP) ou le nicotinamide adénine dinucléotide (NAD); et la glucose-6-phosphate déshydrogénase.

22. Le kit de réactif selon la revendication 13 à 14,
dans lequel la cible de mesure est la créatinine en tant que précurseur,
le kit de réactif doit être utilisé après un traitement de dérivation quantitative de la créatinine en créatine en mettant la créatinine en contact avec la créatinine amidohydrolase (EC 3.5.2.10) en présence d'eau, et comprend:
(a) la créatine kinase;
(b) l'adénosine triphosphate (ATP);
(c) l'inosine diphosphate (IDP);
(d) de la glucokinase dépendante de l'adénosine diphosphate (ADP) (EC 2.7.1.147);
(e) du glucose;
(f) le thionicotinamide adénine dinucléotide phosphate ( thio-NADP), le thionicotinamide adénine dinucléotide (thio-NAD), le nicotinamide adénine dinucléotide phosphate (NADP) ou le nicotinamide adénine dinucléotide (NAD); et
(g) glucose-6-phosphate déshydrogénase.

23. Composition comprenant:
(a) une kinase qui catalyse une réaction directe pour produire un produit phosphorylé à partir d'un substrat de réaction direct d'une kinase et une réaction inverse de celle - ci en présence des coenzymes nucléotidiques, et utilise les coenzymes nucléotides au moins ayant des fragments differents de nucléoside dans la réaction directe et la réaction inverse, respectivement,
(b) une première coenzyme nucléotidique pour la réaction directe; et
(c) une deuxième coenzyme nucléotidique pour la réaction inverse ayant un fragment nucléosidique différent de la première coenzyme nucléotidique,
dans lequel la kinase est la créatine kinase; et le substrat de la réaction directe de la kinase est la créatine.
